(19) Europäisches Patentamt
European Patent Office
Office européen des brevets

(11) Publication number: **0 066 106**
**A1**

(12) **EUROPEAN PATENT APPLICATION**

(21) Application number: 82103897.3

(22) Date of filing: 05.05.82

(51) Int. Cl.³: C 07 C 79/46
C 07 C 149/40, C 07 C 101/52
C 07 C 103/46, C 07 C 125/06
C 07 C 153/09, A 01 N 47/10
A 01 N 37/10, A 01 N 33/22

(30) Priority: 22.05.81 US 266675
15.07.81 US 283402
12.08.81 US 292320
14.09.81 US 301664
13.10.81 US 310663
17.03.82 US 358974

(43) Date of publication of application:
08.12.82 Bulletin 82/49

(84) Designated Contracting States:
BE DE FR GB IT

(71) Applicant: GAF CORPORATION
140 West 51st Street
New York New York 10020(US)

(72) Inventor: Liu, Kou-Chang
11 Drayton Avenue
Wayne New Jersey 07470(US)

(72) Inventor: Brown, Michael J.
11 Cottonwood Drive
Randolph New Jersey 07869(US)

(74) Representative: Patentanwälte Dipl.-Ing. A. Grünecker, Dr.-Ing. H. Kinkeldey, Dr.-Ing. W. Stockmair, Dr. rer. nat. K. Schumann, Dipl.-Ing. P.H. Jakob, Dr. rer. nat. G. Bezold Maximilianstrasse 43
D-8000 München 22(DE)

(54) **New phenoxybenzoates, process for preparing these compounds, compositions containing them and method of use.**

(57) This invention relates to new phenoxybenzoates having high pre- and post- emergence herbicidal activity against indicator weeds, and are particularly effective against broadleaf weeds, such as morning glory. In addition to such effective herbicidal activity, they exhibit an unusual selectivity against important agromonic crops, such as cotton, rice, soybean, corn, wheat and sorghum.

EP 0 066 106 A1

PATENTANWÄLTE
DR. SCHUMANN · JAKOB · DR. BEZOLD · MEISTER

0066106

New Phenoxybenzoates, process for preparing these compounds, compositions containing them and method of use.

This invention relates to certain phenoxybenzoates having excellent selective herbicidal properties. The compounds of the present invention are defined by the formula

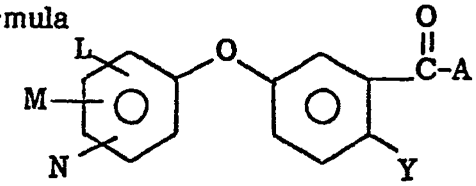

I.

wherein L, M and N are independently hydrogen, hydroxy, halogen, trihalomethyl, nitro, cyano, $C_{1-4}$ alkyl, $C_{1-4}$ alkoxy and 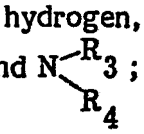 ; Y represents nitro, cyano, or 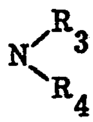 and A is selected from the group of radicals defined by the formulae:

I $-Z(CH_2CH_2Z')_mCH_2CH_2Z''R_7$;

II 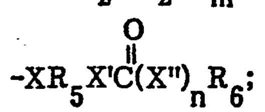

III $-X(CH_2)_nR_6(OH)_p$;

IV 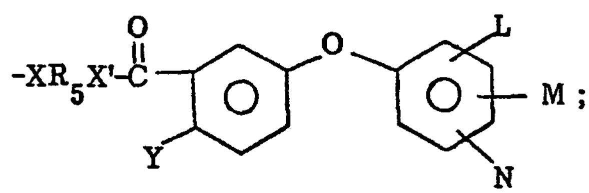

V 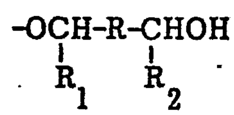 and

VI 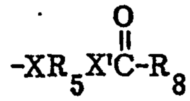

wherein

R is an unsaturated, straight chain or branched aliphatic radical having from 2 to 8 carbon atoms;

$R_1$ and $R_2$ are independently hydrogen or alkyl of 1 to 4 carbon atoms;

$R_3$ and $R_4$ are independently hydrogen, or a saturated or unsaturated straight or branched chain $C_{1-8}$ aliphatic radical optionally substituted with halogen, hydroxy, alkoxy, cyano or nitro;

$\underline{R_5}$ is a saturated or unsaturated, straight chain or branched aliphatic hydrocarbon radical of from 1 to 18 carbon atoms wherein one or more of the $-CH_2-$ groups can be replaced with -O-, -S-, -S-S-, -SO-, $-SO_2-$ or $-NR_4-$ and said hydrocarbon radical is optionally substituted with halogen, trihalomethyl, cyano, aryl, hydroxy, alkoxy, nitro or cycloalkyl having 3 to 6 carbon atoms;

$\underline{R_6}$ is a saturated or unsaturated straight chain or branched aliphatic radical containing from 1 to 8 carbon atoms, optionally substituted with halogen, trihalomethyl, cyano, hydroxy, nitro, acetoxy, alkoxy, thioalkoxy or aryl; an aryl radical optionally substituted with halogen, trihalomethyl, hydroxy, cyano, nitro, alkyl or alkoxy; a cyclic 3-6 membered alkylene ring or 5-6 membered alkenylene ring or benzyl optionally substituted with halogen, trihalomethyl, alkyl, hydroxy, alkoxy or cyano;

$\underline{R_7}$ is hydrogen, $C_{1-4}$ alkyl optionally substituted with halogen, trihalomethyl or cyano or aryl optionally substituted with halogen, trihalomethyl, cyano, $C_{1-4}$ alkoxy or nitro;

$\underline{R_8}$ is $-\overset{O}{\overset{\|}{C}}-(O)_nR_6$, $-R_9-\overset{O}{\overset{\|}{C}}-(OR_9)_n(O)_nR_6$ or $-R_9-O-$ 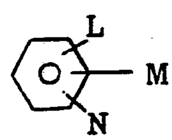 ;

each $\underline{R_9}$ is independently an alkylene diradical having from 1 to 4 carbon atoms;

<u>X, X' and X"</u> are independently -O-, -S- or $-NR_4-$;

<u>Z and Z"</u> are independently, -O- or -S-, Z" additionally can be thiol or $-SO_3-$, and Z' is -S-, -S-S-, -SO- or $-SO_2-$;

<u>p</u> has a value of 2-6; <u>n</u> in each instance, has a value of 0 or 1; and <u>m</u> has a value of 1-6.

Certain diphenylethers have been found effective for control of noxious weeds; however, herbicidal efficacy cannot be predicted from the substituent groups attached to the phenyl rings. More specifically compounds that are closely related frequently have markedly different capabilities for controlling weeds. Often, the diphenylether compounds have overlapping or complimentary areas of activity or selectivity such that combinations of these prior compounds were required for controlling a variety of weeds in a given area. Many of said prior diphenylether compounds are only marginally effective and many leave undesirable residues in the soil as a consequence of low biodegradability or required heavy dosages.

Ideally, a herbicide should provide selective control over an entire growing season after a single application at low dosage rates and should be capable of destroying common weeds as seeds, germinating seeds, seedlings and growing or mature plants. Such herbicides as are recommended for crop treatment should also be non-toxic to desirable vegetation and to the soil. Most beneficially, these herbicides should be dissipatable leaving no harmful residue.

The present herbicidal compounds combine most of these desirable properties; their efficacy being attributable to the active groups defined by A-I through A-VI in formula 1 above. Most active of the present herbicides are those having a sulfur or oxygen atom in the A side chain; and, of these, the side chain defined by formula II exhibits the highest activity in both post emergent and pre-emergent treatments as well as exceptional crop selectivity and high degradability in the soil.

The following specific compounds illustrate the various types of herbicides providing the above benefits; however, these are not to be construed as limiting to the scope of the invention as more properly defined by formula 1.

HERBICIDES REPRESENTATIVE OF GROUP A-I

| Z | Z' | Z" | m | $R_7$ | L | M | N | Y | Name of Compound |
|---|---|---|---|---|---|---|---|---|---|
| S | S | $SO_3$ | 1 | H | 2-Cl | $4-CF_3$ | H | $NO_2$ | 5-{5-[2-Chloro-4-(trifluoromethyl)phenoxy]-2-nitro} phenylcarboxythio-3-thiapentyl sulfonic acid |
| O | S | O | 1 | H | 2-Cl | 4-Cl | 6-Cl | $NO_2$ | 5-Hydroxy-3-thiapentyl[5-(2,4,6-trichlorophenoxy)-2-nitro]benzoate |
| O | S | O | 1 | $CH_3$ | 2-Cl | 4-CN | H | $NO_2$ | 6-Oxa-3-thiaheptyl[5-(2-chloro-4-cyanophenoxy]-2-nitro]benzoate |
| O | S | O | 6 | H | 2-Cl | $4-CF_3$ | H | $NO_2$ | 20-Hydroxy-3,6,9,12,15,18-hexathiaeisosyl 5-[2-chloro-4-(trifluoromethyl)phenoxy]-2-nitrobenzoate |
| O | S | O | 1 | $-CH_2CH_2C{\equiv}N$ | 2-Cl | $4-CF_3$ | H | $NO_2$ | 8-Cyano-6-oxa-3-thiaoctyl 5-[2-chloro-4-(trifluoromethyl)phenoxy]-2-nitrobenzoate |
| O | S | O | 1 | H | $2-CF_3$ | $4-CF_3$ | H | $NO_2$ | 5-Hydroxy-3-thiapentyl 5-[2,4-di(trifluoromethyl-phenoxy]-2-nitrobenzoate |
| S | S | S | 2 | 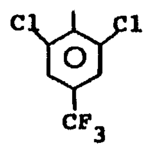 | 2-Cl | $4-CF_3$ | H | $NO_2$ | 8-[2,6-Dichloro-4-(trifluoromethyl)phenoxy]-3,6-dithiaoctyl 5-(2-chloro-4-trifluoromethylphenoxy-2-nitro thiobenzoate |
| O | S | O | 1 | H | $CF_3$ | $CF_3$ | H | $CN_2$ | 5-Hydroxy-3-thiapentyl{5-[2,4-di(trifluoromethyl)phenoxy]-2-cyano} benzoate |

HERBICIDES REPRESENTATIVE OF GROUP A-I

| Z | Z' | Z" | m | $R_7$ | L | M | N | Y | Name of Compound |
|---|---|---|---|---|---|---|---|---|---|
| O | S | O | 1 | H | 2-Cl | 4-$CF_3$ | H | CN | 2-Hydroxy-3-thiapentyl 5-(2-chloro-4-trifluoromethyl phenyl)-2-cyano benzoate |
| O | S | O | 1 | H | Cl | $CF_3$ | H | N< | 2-Hydroxy-3-thiapentyl 5-[2-chloro-4-(trifluoromethyl) phenoxy]-2-(N,N-dimethyl)aminobenzoate |
| S | S | S | 4 | $CH_3$ | Cl | $CF_3$ | H | $NO_2$ | 3,6,9,12,15-Pentathiahexadecyl 5-[2-chloro-4-(trifluoromethyl)phenoxy]-2-nitrothiobenzoate |

HERBICIDES REPRESENTATIVE OF GROUP A-I

| Z | Z' | Z" | m | $R_7$ | L | M | N | Y | Name of Compound |
|---|---|---|---|---|---|---|---|---|---|
| O | S-S | O | 1 | H | 2-Cl | $4-CF_3$ | H | $NO_2$ | 6-Hydroxy-3,4-dithiahexyl {5-[2-chloro-4-(trifluoromethyl)phenoxy]-2-nitro} benzoate |
| O | S | O | 1 | H | 2-Cl | $4-CF_3$ | H | $NO_2$ | 5-Hydroxy-3-thiapentyl {5-[2-chloro-4-(trifluoromethyl)phenoxy]-2-nitro} benzoate |
| O | S-S | O | 1 | H | 2-Cl | 4-Cl | H | $NO_2$ | 6-Hydroxy-3,4-dithiahexyl[5-(2,4-dichlorophenoxy)-2-nitro]benzoate |
| O | $-SO_2-$ | O | 1 | H | 2-Cl | $4-CF_3$ | H | $NO_2$ | 5-Mercapto-3-thiadioxopentyl {5-[2-chloro-4-(trifluoromethyl)phenoxy]-2-nitro} benzoate |
| S | - | S | O | $-CH(CH_3)_2$ | 2-Cl | $4-CF_3$ | H | $NO_2$ | 3-Thia-4-methylpentyl {5-[2-chloro-4-(trifluoromethyl)phenoxy]-2-nitro} thiobenzoate |
| O | S | O | 1 | $-CH_2CH_2Cl$ | 2-Cl | 4-Cl | H | $NO_2$ | 8-Chloro-6-oxa-3-thiaoctyl[5-(2,4-dichlorophenoxy)-2-nitro]benzoate |
| S | S | S | 1 | H | 2-Cl | $4-CF_3$ | H | $NO_2$ | 5-Mercapto-3-thiapentyl {5-[2-chloro-4-(trifluoromethyl)phenoxy]-2-nitro} thiobenzoate |
| O | S | O | 1 | H | 2-Cl | $4-CF_3$ | H | $CN_2$ | 5-Hydroxy-3-thiaoxo-pentyl {5-[2-chloro-4-(trifluoromethyl)phenoxy]-2-cyano} benzoate |
| S | - | $SO_3$ | O | $CH_3$ | 2-Cl | $4-CF_3$ | H | $NO_2$ | Methyl 2-{5-[2-chloro-4-(trifluoromethyl)phenoxy]-2-nitro} phenylcaroxythioethyl sulfonate |

HERBICIDES REPRESENTATIVE OF GROUP A-II

| Compound | L | M | N | X | X' | Y | R | $(X")_nR_6$ |
|---|---|---|---|---|---|---|---|---|
| 2-(Chloroacetoxy)ethyl 5-(2-chloro-4-trifluoromethylphenoxy)-2-nitrobenzoate | 2-Cl | $4-CF_3$ | H | O | O | $NO_2$ | $-CH_2CH_2-$ | $-CH_2Cl$ |
| 2-Acetoxyethyl 5-(2-chloro-4-trifluoromethylphenoxy)-2-nitrobenzoate | 2-Cl | $4-CF_3$ | H | O | O | $NO_2$ | $-CH_2CH_2-$ | $-CH_3$ |
| 2-(Isobutyryloxy)ethyl 5-(2-chloro-4-trifluoromethylphenoxy)-2-nitrobenzoate | 2-Cl | $4-CF_3$ | H | O | O | $NO_2$ | $-CH_2CH_2-$ | 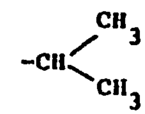 |
| 2-(Cyclopropanecarboxy)ethyl 5-(2-chloro-4-trifluoromethylphenoxy)-2-nitrobenzoate | 2-Cl | $4-CF_3$ | H | O | O | $NO_2$ | $-CH_2CH_2-$ | 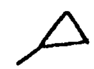 |
| 2-(Acryloyloxy)ethyl 5-(2-chloro-4-trifluoromethylphenoxy)-2-nitrobenzoate | 2-Cl | $4-CF_3$ | H | O | O | $NO_2$ | $-CH_2CH_2-$ | $-CH=CH_2$ |
| 2-(Methoxyacetoxy)ethyl 5-(2-chloro-4-trifluoromethylphenoxy)-2-nitrobenzoate | 2-Cl | $4-CF_3$ | H | O | O | $NO_2$ | $-CH_2CH_2-$ | $-CH_2OCH_3$ |
| 3-Acetoxypropyl 5-(2-chloro-4-trifluoromethylphenoxy)-2-nitrobenzoate | 2-Cl | $4-CF_3$ | H | O | O | $NO_2$ | $-CH_2CH_2CH_2-$ | $-CH_3$ |
| 4-(Chloroacetoxy)butyl 5-(2-chloro-4-trifluoromethylphenoxy)-2-nitrobenzoate | 2-Cl | $4-CF_3$ | H | O | O | $NO_2$ | $-CH_2CH_2CH_2CH_2-$ | $-CH_2Cl$ |

HERBICIDES REPRESENTATIVE OF GROUP A-II

| Compound | L | M | N | X | X' | Y | $R_5$ | $(X")_nR_6$ |
|---|---|---|---|---|---|---|---|---|
| 2-(3-Chlorobenzoyloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | 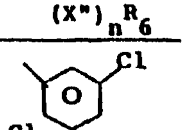 |
| 2-(2,4-Dichlorobenzoyloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | 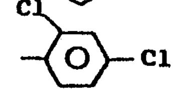 |
| 4-Acetoxy-2-buten-1-yl 5-(2-chloro-4-trifluoromethylphenoxy)-2-nitrobenzoate | 2-Cl | $4-CF_3$ | H | O | O | $NO_2$ | $-CH_2CH{=}CHCH_2-$ | $-CH_3$ |
| 11-Chloroacetoxy-3,6,9-trioxaundecyl 5-(2-chloro-4-trifluoromethylphenoxy)-2-nitrobenzoate | 2-Cl | $4-CF_3$ | H | O | O | $NO_2$ | $-(CH_2CH_2O)_3CH_2CH_2-$ | $-CH_2Cl$ |
| 2-Benzoyloxyethyl 5-(2-chloro-4-trifluoromethylphenoxy)-2-nitrobenzoate | 2-Cl | $4-CF_3$ | H | O | O | $NO_2$ | $-CH_2CH_2-$ | 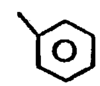 |
| 6-Acetoxy-3,4-dithiahexyl 5-(2-chloro-4-trifluoromethylphenoxy)-2-nitrobenzoate | 2-Cl | $4-CF_3$ | H | O | O | $NO_2$ | $-CH_2CH_2-S-S-CH_2CH_2-$ | $-CH_3$ |
| 2-(3-Amino-2,5-dichlorobenzoyloxy)ethyl 5-(2-chloro-4-trifluoromethylphenoxy)-2-nitrobenzoate | 2-Cl | $4-CF_3$ | H | O | O | $NO_2$ | $-CH_2CH_2-$ | 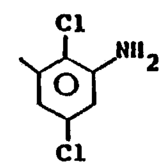 |

HERBICIDES REPRESENTATIVE OF GROUP A-II

| Compound | L | M | N | X | X' | Y | $R_5$ | $(X'')_nR_6$ |
|---|---|---|---|---|---|---|---|---|
| 2-(2-Chloropropionyloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | $\underset{Cl}{CH}-CH_3$ |
| 2-(2-Methylbutyryloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | $-\overset{CH_3}{CH}-CH_2CH_3$ |
| 2-(3,3-Dimethylacryloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | $-CH=C\genfrac{}{}{0pt}{}{CH_3}{CH_3}$ |
| N-(2-Isobutyryloxy)ethyl-N-methyl 5-(2-chloro-4-trifluoromethylphenoxy)-2-nitrobenzamide | 2-Cl | $4-CF_3$ | H | $N-CH_3$ | O | $NO_2$ | $-CH_2CH_2-$ | $-CH\genfrac{}{}{0pt}{}{CH_3}{CH_3}$ |
| N-(2-Acetoxyethyl)-N-isopropyl 5-(2-chloro-4-trifluoromethylphenoxy)-2-nitrobenzoate | 2-Cl | $4-CF_3$ | H | $NCH(CH_3)_2$ | O | $NO_2$ | $-CH_2CH_2-$ | $CH_3$ |
| N-(2-Acetoxyethyl) 5-(2-chloro-4-trifluoromethylphenoxy)-2-nitrobenzamide | 2-Cl | $4-CF_3$ | H | NH | O | $NO_2$ | $-CH_2CH_2-$ | $CH_3$ |
| N,N-[Di-(2-acetoxy) ethyl] 5-(2-chloro-4-trifluoromethylphenoxy)-2-nitrobenzamide | 2-Cl | $4-CF_3$ | H | $N-CH_2CH_2-\overset{O\ O}{C}-CH_3$ | | $NO_2$ | $-CH_2CH_2-$ | $CH_3$ |

HERBICIDES REPRESENTATIVE OF GROUP A-II

| Compound | L | M | N | X | X' | Y | $R_5$ | $(X")_nR_6$ |
|---|---|---|---|---|---|---|---|---|
| 2-(Propionyloxy)ethyl 5-(2-chloro-4-trifluoromethylphenoxy)-2-nitrobenzoate | 2-Cl | $4-CF_3$ | H | O | O | $NO_2$ | $-CH_2CH_2-$ | $-CH_2CH_3$ |
| 2-(Trifluoroacetoxy)ethyl 5-(2-chloro-4-trifluoromethylphenoxy)-2-nitrobenzoate | 2-Cl | $4-CF_3$ | H | O | O | $NO_2$ | $-CH_2CH_2-$ | $-CF_3$ |
| 5-(Chloroacetoxy)-3-oxapentyl 5-(2-chloro-4-trifluoromethylphenoxy)-2-nitrobenzoate | 2-Cl | $4-CF_3$ | H | O | O | $NO_2$ | $-CH_2CH_2OCH_2CH_2-$ | $-CH_2Cl$ |
| 6-(N,N-Dimethylcarbamyloxy)hexyl 5-(2-chloro-4-trifluoromethylphenoxy)-2-nitrobenzoate | 2-Cl | $4-CF_3$ | H | O | O | $NO_2$ | $-(CH_2)_6-$ | 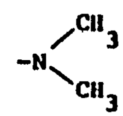 |
| 2-(N,N-Dimethylcarbamyloxy)ethyl 5-(2-chloro-4-trifluoromethylphenoxy)-2-nitrobenzoate | 2-Cl | $4-CF_3$ | H | O | O | $NO_2$ | $-CH_2CH_2-$ | 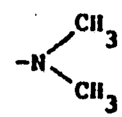 |
| 5-(N,N-Dimethylcarbamyloxy)-3-oxapentyl 5-(2-chloro-4-trifluoromethylphenoxy)-2-nitrobenzoate | 2-Cl | $4-CF_3$ | H | O | O | $NO_2$ | $-CH_2CH_2OCH_2CH_2-$ | 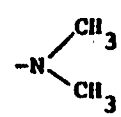 |
| 4-(N,N-Dimethylcarbamyloxy)-2-buten-1-yl 5-(2-chloro-4-trifluoromethylphenoxy)-2-nitrobenzoate | 2-Cl | $4-CF_3$ | H | O | O | $NO_2$ | $-CH_2CH=CHCH_2-$ | 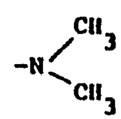 |

HERBICIDES REPRESENTATIVE OF GROUP A-II

| Compound | L | M | N | X | X' | Y | $R_5$ | $(X")_nR_6$ |
|---|---|---|---|---|---|---|---|---|
| 4-(N,N-Dimethylcarbamyloxy) butyl 5-(2,4-dichloro-phenoxy)-2-nitrobenzoate | 2-Cl | 4-Cl | H | O | O | $NO_2$ | $-CH_2CH_2CH_2CH_2-$ | 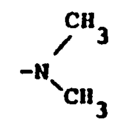 |
| 2-(N,N-Diethylcarbamyloxy) ethyl 5-(2-chloro-4-trifluoromethylphenoxy)-2-nitrobenzoate | 2-Cl | 4-$CF_3$ | H | O | O | $NO_2$ | $-CH_2CH_2-$ | 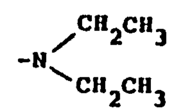 |
| 2-[N-(3-chlorophenyl)carbamyloxy]ethyl 5-(2-chloro-4-trifluoromethylphenoxy)-2-nitrobenzoate | 2-Cl | 4-$CF_3$ | H | O | O | $NO_2$ | $-CH_2CH_2-$ | 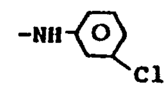 |
| N-2-(N,N-Diethylcarbamyloxy) ethyl-N-propyl 5-(2-chloro-4-trifluoromethylphenoxy)-2-nitrobenzamide | 2-Cl | 4-$CF_3$ | H | $N-CH_2CH_2CH_3$ | O | $NO_2$ | $-CH_2CH_2-$ | 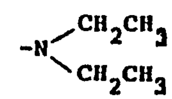 |
| 2-(N,N-Diallylcarbamylamino) ethyl 5-(2-chloro-4-trifluoromethylphenoxy)-2-nitrobenzoate | 2-Cl | 4-$CF_3$ | H | O | NH | $NO_2$ | $-CH_2CH_2-$ | 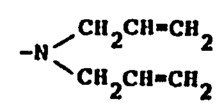 |
| 3-Acetoxy-2,2-dimethylpropyl 5-(2-chloro-4-trifluoromethylphenoxy)-2-nitrobenzoate | 2-Cl | 4-$CF_3$ | H | O | O | $NO_2$ | 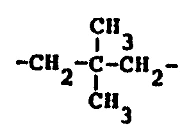 | $CH_3$ |
| N-[2-(Acetylthio)ethyl] 5-(2-chloro-4-trifluoromethylphenoxy)-2-nitrobenzamide | 2-Cl | 4-$CF_3$ | H | NH | S | $NO_2$ | $CH_2CH_2$ | $CH_3$ |
| N-[2-(Acetoxy)ethyl]-N-methyl 5-(2-chloro-4-trifluoromethylphenoxy)-2-nitrobenzamide | 2-Cl | 4-$CF_3$ | H | $NCH_3$ | O | $NO_2$ | $CH_2CH_2$ | $CH_3$ |

HERBICIDES REPRESENTATIVE OF GROUP A-II

| Compound | L | M | N | X | X' | Y | $R_5$ | $(X'')_nR_6$ |
|---|---|---|---|---|---|---|---|---|
| 2-(N,N-Dimethylcarbamylthio) ethyl 5-(2-chloro-4-trifluoromethylphenoxy)-2-nitrobenzamide | 2-Cl | $4-CF_3$ | H | NH | S | $NO_2$ | $-CH_2CH_2-$ | 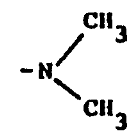 |
| 2-[N-(N,N-Diethylcarbamyl)amino] ethyl 5-(2-chloro-4-trifluoromethylphenoxy)-2-nitrobenzoate | 2-Cl | $4-CF_3$ | H | O | NH | $NO_2$ | $-CH_2CH_2-$ | 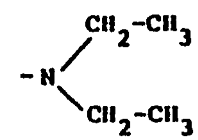 |
| 2-(N-Acetylamino) ethyl 5-(2-chloro-4-trifluoromethylphenoxy)-2-nitrobenzoate | 2-Cl | $4-CF_3$ | H | O | NH | $NO_2$ | $-CH_2CH_2-$ | $CH_3$ |
| 2-(3-Cyanopropionyloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | $CH_2CH_2-CN$ |
| 2-(3-Chloropropionyl)ethyl 5-(2-chloro-4-trifluoromethylphenoxy)-2-nitrobenzoate | 2-Cl | $4-CF_3$ | H | O | O | $NO_2$ | $-CH_2CH_2-$ | $CH_2CH_2Cl$ |
| 2-(Ethynylacetoxy)ethyl 5-(2-chloro-4-trifluoromethylphenoxy)-2-nitrobenzoate | 2-Cl | $4-CF_3$ | H | O | O | $NO_2$ | $-CH_2CH_2-$ | $CH_2-C\equiv CH$ |
| 2-(3-Nitropropionyloxy) ethyl 5-(2-chloro-4-trifluoromethylphenoxy)-2-nitrobenzoate | 2-Cl | $4-CF_3$ | H | O | O | $NO_2$ | $-CH_2CH_2-$ | $CH_2CH_2NO_2$ |

HERBICIDES REPRESENTATIVE OF GROUP A-II

| Compound | L | M | N | X | X' | Y | $R_5$ | $(X'')_nR_6$ |
|---|---|---|---|---|---|---|---|---|
| 2-(3,3,3-Trifluoroprop-ionyloxy)ethyl 5-(2-chloro-4-trifluoromethyl-phenoxy)-2-nitrobenzoate | 2-Cl | $4-CF_3$ | H | O | O | $NO_2$ | $-CH_2CH_2-$ | $CH_2CF_3$ |
| 2-(Thiomethoxyacetoxy) ethyl 5-(2-chloro-4-tri-fluoromethylphenoxy)-2-nitrobenzoate | 2-Cl | $4-CF_3$ | H | O | O | $NO_2$ | $-CH_2CH_2-$ | $CH_2SCH_3$ |
| 2-[3-(N,N-Dimethylamino)pro-pionyloxy]ethyl 5-(2-chloro-4-trifluoromethylphenoxy) -2-nitrobenzoate | 2-Cl | $4-CF_3$ | H | O | O | $NO_2$ | $-CH_2CH_2-$ | 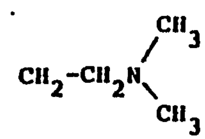 |
| 2-(Cyclohexylacetoxy) ethyl 5-(2-chloro-4-trifluoromethylphenoxy) -2-nitrobenzoate | 2-Cl | $4-CF_3$ | H | O | O | $NO_2$ | $-CH_2CH_2-$ | 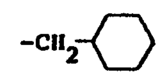 |
| 2-(3-Hydroxypropionyloxy) ethyl 5-(2-chloro-4-tri-fluoromethylphenoxy)-2-nitrobenzoate | 2-Cl | $4-CF_3$ | H | O | O | $NO_2$ | $-CH_2CH_2-$ | $CH_2CH_2OH$ |
| 2-(Phenylacetoxy)ethyl 5-(2-chloro-4-trifluoro-methylphenoxy)-2-nitrobenzoate | 2-Cl | $4-CF_3$ | H | O | O | $NO_2$ | $-CH_2CH_2-$ | 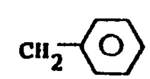 |

HERBICIDES REPRESENTATIVE OF GROUP A-II

| Compound | L | M | N | X | X' | Y | $R_5$ | $(X'')_n$ | $R_6$ |
|---|---|---|---|---|---|---|---|---|---|
| N-methyl-N-(2-acetoxyethyl)5-(2-chloro-4-trifluoromethylphenoxy)-2-nitrobenzoamide | 2-Cl | $4-CF_3$ | H | $NCH_3$ | O | $NO_2$ | $-CH_2-CH_2-$ | | $-CH_3$ |
| 3-Chloroacetoxy-2-(hydroxymethyl)propyl 5-(2-chloro-4-trifluoromethylphenoxy)-2-nitrobenzoate | 2-Cl | $4-CF_3$ | H | O | O | $NO_2$ | $-CH_2-CH-CH_2-$<br>\|<br>$CH_2OH$ | | $-CH_2Cl$ |
| 2-Chloromethyl-3-isobutynyl-oxypropyl 5-(2-chloro-4-trifluoromethylphenoxy)-2-nitrobenzoate | 2-Cl | $4-CF_3$ | H | O | O | $NO_2$ | $-CH_2-CH_2-CH_2-$<br>\|<br>$CH_2Cl$ | | $CH_3$<br>$-CH$<br>$CH_3$ |
| 3-Propionyloxy-2,2-(dimethyl) propyl 5-(2-chloro-4-trifluoromethylphenoxy)-2-nitrobenzoate | 2-Cl | $4-CF_3$ | H | O | O | $NO_2$ | $CH_3$<br>\|<br>$-CH_2-C-CH_2-$<br>\|<br>$CH_3$. | | $-CH_2CH_3$ |
| 2-Chloroacetoxymethyl-4-cyanobutyl 5-(2,4,6-trichlorophenoxy)-2-nitrobenzoate | 2-Cl | 4-Cl | 6-Cl | O | O | $NO_2$ | $-CH_2-CH-CH_2-$<br>\|<br>$CH_2CH_2CN$ | | $-CH_2Cl$ |
| 3-Fluoroacetoxy-2-(methoxymethyl)propyl 5-(2,4-dichlorophenoxy)-2-nitrobenzoate | 2-Cl | 4-Cl | H | O | O | $NO_2$ | $-CH_2-CH-CH_2-$<br>\|<br>$CH_2OCH_3$ | | $-CH_2F$ |
| 2-(Chloroacetoxy)ethyl 2-cyano-5-(2,6-dichloro-4-trifluoromethylphenoxy)benzoate | 2-Cl | $4-CF_3$ | 6-Cl | O | O | CN | $-CH_2CH_2-$ | | $-CH_2Cl$ |

HERBICIDES REPRESENTATIVE OF GROUP A-II

| Compound | L | M | N | X | X' | Y | $R_5$ | $(X'')_nR_6$ |
|---|---|---|---|---|---|---|---|---|
| 2-(Chloroacetoxy)ethyl 5-(2-chloro-4-trifluoromethylphenoxy)-2-cyanobenzoate | 2-Cl | $4-CF_3$ | H | O | O | CN | $-CH_2-CH_2-$ | $-CH_2Cl$ |
| 2-(Chloroacetoxy)ethyl-2-amino-5-(2-chloro-4-trifluoromethylphenoxy) benzoate | 2-Cl | $4-CF_3$ | H | O | O | $-NH_2$ | $-CH_2CH_2-$ | $-CH_2Cl$ |
| 2-(Chloroacetoxy)ethyl 5-(2-chloro-4-trifluoromethylphenoxy)-2-(N,N-dimethylamino)benzoate | 2-Cl | $4-CF_3$ | H | O | O | 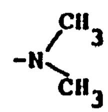 | $-CH_2CH_2-$ | $-CH_2Cl$ |
| 2-(N,N-Dimethylcarbamoyl amino)ethyl 5-(2-chloro-4-trifluoromethylphenoxy) -2-nitrobenzoate | 2-Cl | $4-CF_3$ | H | O | NH | $NO_2$ | $-CH_2CH_2-$ | 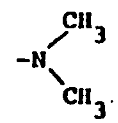 |
| N-[2-(N,N-Diallylcarbamyloxy)ethyl] 5-(2-chloro-4-trifluoromethylphenoxy)-2-nitrobenzamide | 2-Cl | $4-CF_3$ | H | NH | O | $NO_2$ | $-CH_2CH_2-$ | 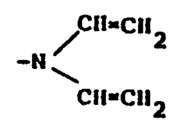 |
| 2-[N-Methyl-N-(N,N-dimethylcarbamyl)amino] 5-(2-chloro-4-trifluoromethylphenoxy)-2-nitrobenzoate | 2-Cl | $4-CF_3$ | H | O | $NCH_3$ | $NO_2$ | $-CH_2CH_2-$ | 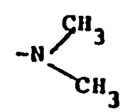 |

HERBICIDES REPRESENTATIVE OF GROUP A-II

| Compound | L | M | N | X | X' | Y | $R_5$ | $(X'')_nR_6$ |
|---|---|---|---|---|---|---|---|---|
| 2-(N,N-Dimethylcarbamylthio)ethyl 5-(2-chloro-4-trifluoromethylphenoxy)-2-nitrothiobenzoate | 2-Cl | 4-$CF_3$ | H | S | S | $NO_2$ | $-CH_2CH_2-$ | 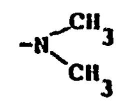 |
| N-{2-(S-Ethylthiocarboxyamino)ethyl} 5-(2-chloro-4-(trifluoromethylphenoxy)-2-nitrobenzamide | 2-Cl | 4-$CF_3$ | H | NH | NH | $NO_2$ | $-CH_2CH_2-$ | $-S-CH_2CH_3$ |
| N-[2-Ethoxycarboxyamino)ethyl] 5-(2-chloro-4-trifluoromethylphenoxy)-2-nitrobenzamide | 2-Cl | 4-$CF_3$ | H | NH | NH | $NO_2$ | $-CH_2CH_2-$ | $-OCH_2CH_3$ |
| N-Methyl-N-[2-(N,N-diethylcarbamyloxy)ethyl] 5-(2-chloro-4-trifluoromethylphenoxy)-2-nitrobenzamide | 2-Cl | 4-$CF_3$ | H | $NCH_3$ | O | $NO_2$ | $-CH_2CH_2-$ | 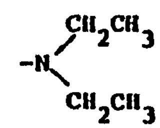 |
| 2-(N,N-dimethylcarbamylthio)ethyl 5-(2,4,6-trichlorophenoxy)-2-nitrothiobenzoate | 2-Cl | 4-Cl | 6-Cl | S | S | $NO_2$ | $-CH_2CH_2-$ | 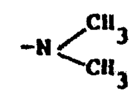 |
| 2-(N,N-Dimethylcarbamyloxy)ethyl 2-cyano -5-(2-chloro-4-trifluoromethylphenoxy)benzoate | 2-Cl | 4-$CF_3$ | H | O | O | CN | $-CH_2CH_2-$ | 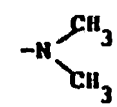 |

HERBICIDES REPRESENTATIVE OF GROUP A-II

| Compound | L | M | N | X | X' | Y | $R_5$ | $(X")_nR_6$ |
|---|---|---|---|---|---|---|---|---|
| 2-(N,N-Diethylcarbamyl-oxy)ethyl 5-(chloro-4-tri-fluoromethylphenoxy)-2-cyanobenzoate | 2-Cl | 4-$CF_3$ | H | O | O | CN | $-CH_2CH_2-$ | 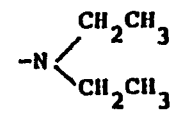 |
| 2-(Acetylthio)ethyl 5-(2-chloro-4-trifluoromethyl-phenoxy)-2-nitrothiobenzoate | 2-Cl | 4-$CF_3$ | H | S | S | $NO_2$ | $-CH_2CH_2-$ | $-CH_3$ |
| 2-Acetoxyethyl 5-(2-chloro-4-trifluoromethylphenoxy)-2-nitrothiobenzoate | 2-Cl | 4-$CF_3$ | H | S | O | $NO_2$ | $-CH_2CH_2-$ | $-CH_3$ |
| 2-(Acetylthio)ethyl 5-(2-chloro-4-trifluoromethyl-phenoxy)-2-nitrobenzoate | 2-Cl | 4-$CF_3$ | H | O | S | $NO_2$ | $-CH_2CH_2-$ | $-CH_3$ |
| 2-Acetadmidoethyl 5-(2-chloro-4-trifluoromethyl-phenoxy)-2-nitrothiobenzoate | 2-Cl | 4-$CF_3$ | H | S | NH | $NO_2$ | $-CH_2CH_2-$ | $-CH_3$ |
| N-[2-(Acetylthio)ethyl] 5-(2-chloro-4-trifluoro-methylphenoxy)-2-nitro-benzoamide | 2-Cl | 4-$CF_3$ | H | NH | S | $NO_2$ | $-CH_2CH_2-$ | $-CH_3$ |
| N-Methyl-N-[2-(Acetylthio)ethyl]5-(2-chloro-4-tri-fluoromethylphenoxy)-2-nitrobenzamide | 2-Cl | 4-$CF_3$ | H | $NCH_3$ | S | $NO_2$ | $-CH_2CH_2-$ | $-CH_3$ |

HERBICIDES REPRESENTATIVE OF GROUP A-II

| Compound | L | M | N | X | X' | Y | $R_5$ | $(X'')_nR_6$ |
|---|---|---|---|---|---|---|---|---|
| 2-(N-Methylacetamido) ethyl 5-(2-chloro-4-trifluoromethylphenoxy)-2-nitrothiobenzoate | 2-Cl | $4-CF_3$ | H | S | $NCH_3$ | $NO_2$ | $-CH_2CH_2-$ | $-CH_3$ |
| 2-Acetamidoethyl 5-(2-chloro-4-trifluoromethylphenoxy)-2-nitrobenzamide | 2-Cl | $4-CF_3$ | H | O | NH | $NO_2$ | $-CH_2CH_2-$ | $-CH_3$ |
| 2-(N-methylacetamido)ethyl 5-(2-chloro-4-trifluoromethylphenoxy)-2-nitrobenzoate | 2-Cl | $4-CF_3$ | H | O | $NCH_3$ | $NO_2$ | $-CH_2CH_2-$ | $-CH_3$ |
| 2-(Chloroacetoxy)ethyl 2-nitro-5-[di-(trifluoromethyl)phenoxy]benzoate | $2-CF_3$ | $4-CF_3$ | H | O | O | $NO_2$ | $-CH_2CH_2-$ | $-CH_2Cl$ |
| 2-Acetoxyethyl 5-(4-chlorb-2-trifluoromethylphenoxy)-2-nitrobenzoate | $2-CF_3$ | 4-Cl | H | O | O | $NO_2$ | $-CH_2CH_2-$ | $-CH_3$ |
| 2-(Chloroacetoxy) ethyl 2-nitro-5-(2-amino-4-trifluoromethylphenoxy)benzoate | $2-NH_2$ | $4-CF_3$ | H | O | O | $NO_2$ | $-CH_2CH_2-$ | $-CH_2Cl$ |
| 2-(Cyclopropanecarboxythio) ethyl 5-(2-chloro-4-trifluoromethylphenoxy)-2-nitrothiobenzoate | $2-CF_3$ | $4-NO_2$ | H | S | S | $NO_2$ | $-CH_2CH_2-$ | 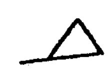 |

HERBICIDES REPRESENTATIVE OF GROUP A-II

| Compound | L | M | N | X | X' | Y | $R_5$ | $(X'')_n R_6$ |
|---|---|---|---|---|---|---|---|---|
| 2-(Chloroacetoxy)ethyl 5-(5-chloro-2-cyano-4-trifluoromethylphenoxy)-2-nitrobenzoate | 2-CN | $4-CF_3$ | 5-Cl | O | O | $NO_2$ | $-CH_2CH_2-$ | $-CH_2Cl$ |
| 2-Acetoxyethyl 5-(2-chloro-4-N,N-dimethylamino-5-methylphenoxy)-2-nitrobenzoate | 2-Cl | 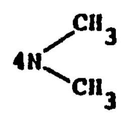 | $5-CH_3$ | O | O | $NO_2$ | $-CH_2CH_2-$ | $-CH_3$ |
| 2-(Chloroacetoxy)ethyl 5-(2-chloro-4-methoxyphenoxy)-2-nitrobenzoate | 2-Cl | $4OCH_3$ | H | O | O | $NO_2$ | $-CH_2CH_2-$ | $-CH_2Cl$ |
| 2-(Dichloroacetoxy)ethyl 5-(2-chloro-5-cyano-4-trifluoromethylphenoxy)-2-nitrobenzoate | 2-Cl | $4-CF_3$ | CN | O | O | $NO_2$ | $-CH_2CH_2-$ | $-CHCl_2$ |

HERBICIDES REPRESENTATIVE OF GROUP A-III

| Compound | L | M | N | X | n | Y | $R_6(OH)_p$* |
|---|---|---|---|---|---|---|---|
| 2,2-Bis(hydroxymethyl)propyl 5-(2-chloro-4-trifluoromethyl phenoxy)-2-nitrobenzoate | 2-Cl | 4-$CF_3$ | H | O | 1 | $NO_2$ | 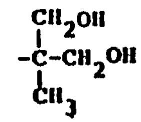 |
| (2,3-Dihydroxypropyl) 5-(2-chloro-4-trifluoromethyl phenoxy)-2-nitrobenzoate | 2-Cl | 4-$CF_3$ | H | O | 1 | $NO_2$ | 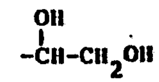 |
| (2,3,4-Trihydroxybutyl) 5-(2-chloro-4-trifluoromethylphenoxy)-2-nitrobenzoate | 2-Cl | 4-$CF_3$ | H | O | 1 | $NO_2$ | 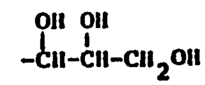 |
| 3-Hydroxy-2,2-di(hydroxymethyl) propyl 5-(2-chloro-4-trifluoromethyl phenoxy)-2-nitrobenzoate | 2-Cl | 4-$CF_3$ | H | O | 1 | $NO_2$ | 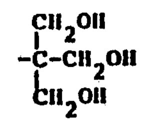 |
| (2,3,4,5-Tetrahydroxypentyl) 5-(2-chloro-4-trifluoromethyl phenoxy)-2-nitrobenzoate | 2-Cl | 4-$CF_3$ | H | O | 1 | $NO_2$ | 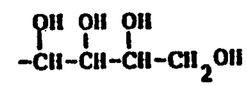 |
| (4-Cyano-2,3-dihydroxybutyl) 5-(2-chloro-4-trifluoromethyl phenoxy)-nitrobenzoate | 2-Cl | 4-$CF_3$ | H | O | 1 | $NO_2$ | 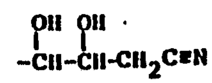 |

* $(OH)_p$ designates the number of -OH groups in the $R_6$ radical, however $R_6$ itself can be mono- or polyhydroxylated. Thus, the terminal group $R_6(OH)_p$ can also be defined simply as $R_6$ where $R_6$ is a polyhydroxylated radical.

## HERBICIDES REPRESENTATIVE OF GROUP A-III

| Compound | L | M | N | X | n | Y | $R_6(OH)_p$ |
|---|---|---|---|---|---|---|---|
| (3-Chloro-2,4,5-trihydroxypentyl) 5-(2-chloro-4-trifluoromethyl phenoxy)-2-nitrobenzoate | 2-Cl | $4-CF_3$ | H | O | 1 | $NO_2$ | 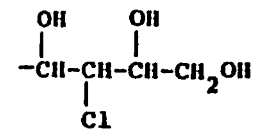 |
| (2,3,4-Trihydroxy-5-methoxypentyl) 5-(2-chloro-4-trifluoromethyl phenoxy)-2-nitrobenzoate | 2-Cl | $4-CF_3$ | H | O | 1 | $NO_2$ | 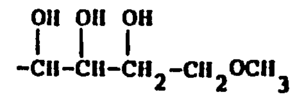 |
| (4-Acetoxy-2,3-dihydroxybutyl) 5-(2-chloro-4-trifluoromethyl phenoxy)-2-nitrobenzoate | 2-Cl | $4-CF_3$ | H | O | 1 | $NO_2$ | 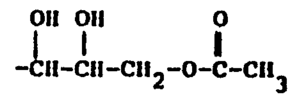 |
| (2,3-Dihydroxy-4-trifluoromethylbutyl) 5-(2-chloro-4-trifluoromethyl phenoxy)-2-nitrobenzoate | 2-Cl | $4-CF_3$ | H | O | 1 | $NO_2$ | 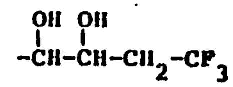 |
| (2,3-Dihydroxy-4-nitrobutyl) 5-(2-chloro-4-trifluoromethyl phenoxy)-2-nitrobenzoate | 2-Cl | $4-CF_3$ | H | O | 1 | $NO_2$ | 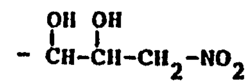 |
| (2,3,4,5,6-Pentahydroxycyclohexyl) 5-(2-chloro-4-trifluoromethyl phenoxy)-2-nitrobenzoate | 2-Cl | $4-CF_3$ | H | O | 0 | $NO_2$ | 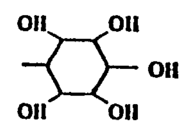 |

## HERBICIDES REPRESENTATIVE OF GROUP A-III

| Compound | L | M | N | X | n | Y | $R_6(OH)_p$ |
|---|---|---|---|---|---|---|---|
| (3,5-Dihydroxyphenyl) 5-(2-chloro-4-trifluoromethyl phenoxy)-2-nitrobenzoate | 2-Cl | $4-CF_3$ | H | O | 0 | $NO_2$ | 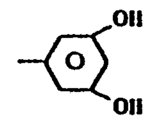 |
| (2,4,6-Trihydroxybenzyl) 5-(2-chloro-4-trifluoromethyl phenoxy)-2-nitrobenzoate | 2-Cl | $4-CF_3$ | H | O | 1 | $NO_2$ | 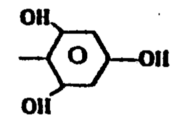 |
| N-(2,3-Dihydroxy)propyl 5-(2-chloro-4-trifluoromethyl phenoxy)-2-nitrobenzamide | 2-Cl | $4-CF_3$ | H | NH | 1 | $NO_2$ | 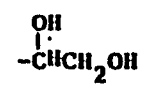 |
| N-(2,3-Dihydroxy)propyl-N-methyl 5-(2-chloro-4-trifluoromethyl phenoxy)-2-nitrobenzamide | 2-Cl | $4-CF_3$ | H | $NCH_3$ | 1 | $NO_2$ | 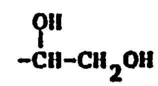 |
| 2,2-Di-(hydroxymethyl)propyl 5-(2-chloro-4-trifluoromethyl-phenoxy)-2-nitrothiobenzoate | 2-Cl | $4-CF_3$ | H | S | 1 | $NO_2$ | 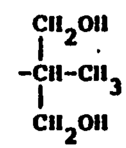 |

HERBICIDES REPRESENTATIVE OF GROUP A-III

| Compound | L | M | N | X | n | Y | $R_6(OH)_p$ |
|---|---|---|---|---|---|---|---|
| (2,3,4-Trihydroxybutyl) 2-cyano 5-(2-chloro-4-trifluoromethyl phenoxy)benzoate | 2-Cl | $4-CF_3$ | H | O | 1 | CN | $-\overset{OH}{CH}-\overset{OH}{CH}-CH_2OH$ |
| (2,3-Dihydroxypropyl) 5-(2-chloro-4-trifluoromethylphenoxy)-2-cyanobenzoate | 2-Cl | $4-CF_3$ | H | O | 1 | CN | $-\overset{OH}{CH}-CH_2OH$ |
| (2,3,4-Trihydroxybutyl) 2-amino-5-(2-chloro-4-trifluoromethyl phenoxy)benzoate | 2-Cl | $4-CF_3$ | H | O | 1 | $NH_2$ | $-\overset{OH}{CH}-\overset{OH}{CH}-CH_2OH$ |
| (2,3,4-Trihydroxybutyl) 2-(N,N-dimethyl)amino-5-(2-chloro-4-trifluoromethyl phenoxy)benzoate | 2-Cl | $4-CF_3$ | H | O | 1 | $N(CH_3)_2$ | $-\overset{OH}{CH}-\overset{OH}{CH}-CH_2OH$ |
| (2,3-Dihydroxypropyl) 5-(2,4,6-trichlorophenoxy)benzoate | 2-Cl | 4-Cl | 6-Cl | O | 1 | $NO_2$ | $-\overset{OH}{CH}-CH_2OH$ |
| (2,3-Dihydroxypropyl) 5-[2,4-di-(trifluoromethyl)phenoxy]-2-nitrobenzoate | $2-CF_3$ | $4-CF_3$ | H | O | 1 | $NO_2$ | $-\overset{OH}{CH}-CH_2OH$ |
| (2,3-Dihydroxypropyl) 5-(2-methyl-6-nitrophenoxy)-2-nitrobenzoate | $2-CH_3$ | H | $6-NO_2$ | O | 1 | $NO_2$ | $-\overset{OH}{CH}-CH_2OH$ |
| (2,3-Dihydroxypropyl) 5-(2-chloro-3-cyano-4-trifluoromethylphenoxy)-2-nitrobenzoate | 2-Cl | 3-CN | $4-CF_3$ | O | 1 | $NO_2$ | $-\overset{OH}{CH}-CH_2OH$<br>$-\overset{OH}{CH}-CH_2OH$ |

HERBICIDES REPRESENTATIVE OF GROUP A-IV

| Compound | L | M | N | X | X' | Y | $R_5$ |
|---|---|---|---|---|---|---|---|
| 1,5-Bis-[5-(2-chloro-4-trifluoromethylphenoxy)-2-nitro-benzoyloxy]-3-oxapentane | 2-Cl | 4-$CF_3$ | H | O | O | $NO_2$ | $-CH_2CH_2OCH_2CH_2-$ |
| 1,6-Bis-[5-(2-chloro-4-trifluoromethylphenoxy)-2-nitro-benzoyloxy]-3,4-dithiahexane | 2-Cl | 4-$CF_3$ | H | O | O | $NO_2$ | $-CH_2CH_2SSCH_2CH_2-$ |
| 1,4-Bis-[5-(2-chloro-4-trifluoromethylphenoxy-2-nitrobenzoyloxy]butane | 2-Cl | 4-$CF_3$ | H | O | O | $NO_2$ | $-CH_2CH_2CH_2CH_2-$ |
| 1,6-Bis-[5-(2-chloro-4-trifluoromethylphenoxy)-2-nitro-benzoyloxy]hexane | 2-Cl | 4-$CF_3$ | H | O | O | $NO_2$ | $-CH_2CH_2CH_2CH_2CH_2CH_2-$ |
| 1,11-Bis-[5-(2-chloro-4-trifluoromethylphenoxy)-2-nitro-benzoyloxy]-3,6,9-trioxaundecane | 2-Cl | 4-$CF_3$ | H | O | O | $NO_2$ | $-CH_2CH_2OCH_2CH_2OCH_2CH_2OCH_2CH_2-$ |
| 1,2-Bis-[5-(2-chloro-4-trifluoromethylphenoxy)-2-nitro-benzoyloxy]ethane | 2-Cl | 4-$CF_3$ | H | O | O | $NO_2$ | $-CH_2CH_2-$ |
| 1,3-Bis-[5-(2-chloro-4-trifluoromethylphenoxy)-2-nitro-benzoyloxy]propane | 2-Cl | 4-$CF_3$ | H | O | O | $NO_2$ | $-CH_2CH_2CH_2-$ |
| 1,10-Bis-[5-(2-chloro-4-trifluoromethylphenoxy)-2-nitro-benzoyloxy]decane | 2-Cl | 4-$CF_3$ | H | O | O | $NO_2$ | $-(CH_2)_{10}-$ |

HERBICIDES REPRESENTATIVE OF GROUP A-IV

| Compound | L | M | N | X | X' | Y | $R_5$ |
|---|---|---|---|---|---|---|---|
| 1,5-Bis-[5-(2-chloro-4-trifluoromethylphenoxy)-2-nitrobenzoyloxy]-3-thiapentane | 2-Cl | $4-CF_3$ | H | O | O | $NO_2$ | $-CH_2CH_2SCH_2CH_2-$ |
| N-{2-[5-(2-chloro-4-trifluoromethylphenoxy)-2-nitrothiobenzoyloxy]} ethyl 5-(2-chloro-4-trifluoromethylphenoxy)-2-nitrobenzamide | 2-Cl | $4-CF_3$ | H | NH | S | $NO_2$ | $-CH_2CH_2-$ |
| 1,2-Bis-[5-(2-chloro-4-trifluoromethylphenoxy)-2-nitrothiobenzoyloxy]ethane | 2-Cl | $4-CF_3$ | H | S | S | $NO_2$ | $-CH_2CH_2-$ |
| 1,4-Bis-[5-(2-chloro-4-trifluoromethylphenoxy)-2-nitrobenzoyloxy]-2-butene | 2-Cl | $4-CF_3$ | H | O | O | $NO_2$ | $-CH_2CH=CHCH_2-$ |
| 1,4-Bis-[5-(2-chloro-4-trifluoromethylphenoxy)-2-nitrobenzoyloxy]-2-butyne | 2-Cl | $4-CF_3$ | H | O | O | $NO_2$ | $-CH_2C{\equiv}CCH_2-$ |
| 1,8-Bis-[5-(2-chloro-4-trifluoromethylphenoxy)-2-nitrobenzoyloxy]-3,5-dioxaoctane | 2-Cl | $4-CF_3$ | H | O | O | $NO_2$ | $-CH_2CH_2OCH_2CH_2OCH_2CH_2-$ |
| N,N'-Bis[5-(2-chloro-4-trifluoromethylphenoxy)-2-nitrobenzoyl]-1,2-diaminoethane | 2-Cl | $4-CF_3$ | H | NH | NH | $NO_2$ | $-CH_2CH_2-$ |
| 2,4-Bis[5-(2-chloro-4-trifluoromethylphenoxy)-2-nitrobenzoyloxy] hexane | 2-Cl | $4-CF_3$ | H | O | O | $NO_2$ | $-CH(CH_3)-CH_2-CH_2-CH(CH_3)-$ |

HERBICIDES REPRESENTATIVE OF GROUP A-IV

| Compound | L | M | N | X | X' | Y | $R_5$ |
|---|---|---|---|---|---|---|---|
| 1,2-Bis-[5-(2-chloro-4-trifluoromethylphenoxy)-2-nitrobenzoyloxy]-3-chloropropane | 2-Cl | $4-CF_3$ | H | O | O | $NO_2$ | $-CH_2-CH-$ $CH_2Cl$ |
| 1,3-Bis-[5-(2-chloro-4-trifluoromethylphenoxy)-2-nitrobenzoyloxy]-2-hydroxypropane | 2-Cl | $4-CF_3$ | H | O | O | $NO_2$ | $-CH_2-CH-CH_2-$ OH |
| 1,3-Bis-[5-(2-chloro-4-trifluoromethylphenoxy)-2-nitrobenzoyloxy]-2-methoxypropane | 2-Cl | $4-CF_3$ | H | O | O | $NO_2$ | $-CH_2-CH-CH_2-$ $OCH_3$ |
| 1,6-Bis-[5-(2-chloro-4-trifluoromethylphenoxy)-2-nitrobenzoyloxy] 2-cyanohexane | 2-Cl | $4-CF_3$ | H | O | O | $NO_2$ | $-CH_2-CH-CH_2CH_2-CH_2-CH_2-$ CN |
| 1,2-Bis-[5-(2-chloro-4-trifluoromethylphenoxy)-2-nitrobenzoyloxy]-3-nitropropane | 2-Cl | $4-CF_3$ | H | O | O | $NO_2$ | $-CH_2-CH-$ $CH_2NO_2$ |
| N-2-[5-(2-chloro-4-trifluoromethylphenoxy)-2-nitrobenzoyloxy]ethyl 5-(2-chloro-4-trifluoromethylphenoxy)-2-nitrobenzamide | 2-Cl | $4-CF_3$ | H | NH | O | $NO_2$ | $-CH_2CH_2-$ |
| N-Ethyl-N-2-[5-(2-chloro-4-trifluoromethylphenoxy)-2-nitrobenzoyloxy]ethyl 5-(2-chloro-4-trifluoromethylphenoxy)-2-nitrobenzamide | 2-Cl | $4-CF_3$ | H | $NCH_2CH_3$ | O | $NO_2$ | $-CH_2-CH_2-$ |

HERBICIDES REPRESENTATIVE OF GROUP A-IV

| Compound | L | M | N | X | X' | Y | $R_5$ |
|---|---|---|---|---|---|---|---|
| 2-[5-(2-chloro-4-trifluoromethylphenoxy)-2-nitrobenzoyloxy]ethyl 5-(2-chloro-4-trifluoromethylphenoxy)-2-nitrothiobenzoate | 2-Cl | 4-$CF_3$ | H | S | O | $NO_2$ | $-CH_2CH_2-$ |
| 1,2-Bis[5-(2,4-dichlorophenoxy)-2-nitrobenzoyloxy]ethane | 2-Cl | 4-Cl | H | O | O | $NO_2$ | $-CH_2CH_2-$ |
| 1,4-Bis-[5-(2,4,6-trichlorophenoxy)-2-nitrobenzoyloxy]-2-butene | 2-Cl | 4-Cl | 6-Cl | O | O | $NO_2$ | $-CH_2CH{=}CHCH_2-$ |
| 1,2-Bis[5-(2,6-dichloro-4-trifluoromethylphenoxy)-2-nitrobenzoyloxy]ethane | 2-Cl | 4-$CF_3$ | 6-Cl | O | O | $NO_2$ | $-CH_2CH_2-$ |
| 1,2-Bis-[5-(2-chloro-4-methoxy phenoxy)-2-nitrobenzoyloxy] ethane | 2-Cl | 4$OCH_3$ | H | O | O | $NO_2$ | $-CH_2CH_2-$ |
| 1,2-Bis-[5-(2-chloro-4-cyano phenoxy)-2-nitrobenzoyloxy] ethane | 2-Cl | 4CN | H | O | O | $NO_2$ | $-CH_2CH_2-$ |
| 1,2-Bis-[5-(5-amino-2-chloro-4-methylphenoxy)-2-nitrobenzoyloxy]ethane | 2-Cl | 4$CH_3$ | 5-$NH_2$ | O | O | $NO_2$ | $-CH_2CH_2-$ |

HERBICIDES REPRESENTATIVE OF GROUP A-IV

| Compound | L | M | N | X | X' | Y | $R_5$ |
|---|---|---|---|---|---|---|---|
| 1,2-Bis-[5-(2-chloro-4-trifluoromethylphenoxy)-2-cyanobenzoyloxy]ethane | 2-Cl | $4-CF_3$ | H | O | O | CN | $-CH_2CH_2-$ |
| 1,2-Bis-[2-amino-5-(2-chloro-4-trifluoromethylphenoxy)benzoyloxy]ethane | 2-Cl | $4-CF_3$ | H | O | O | $NH_2$ | $-CH_2CH_2-$ |
| 1,2-Bis-[2-(N,N-dimethylamino)-5-(2-chloro-4-trifluoromethylphenoxy)benzoyloxy]ethane | 2-Cl | $4-CF_3$ | H | O | O | 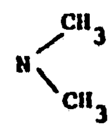 | $-CH_2CH_2-$ |
| 1,2-Bis-[2-cyano-5-(2,6-dichloro-4-trifluoromethylphenoxy)benzoyloxy]ethane | 2-Cl | $4-CF_3$ | Cl | O | O | CN | $-CH_2CH_2-$ |
| 1,2-Bis-[5-(2-chloro-4-trifluoromethylphenoxy)benzoyloxy]ethane | 2-Cl | $4-CF_3$ | H | O | O | H | $-CH_2-CH_2-$ |

HERBICIDES REPRESENTATIVE OF GROUP A-V

| Compound | L | M | N | Y | $R_1$ | $R_2$ | R |
|---|---|---|---|---|---|---|---|
| 4-Hydroxy-2-buten-1-yl 5-(2-chloro-4-trifluoromethylphenoxy)-2-nitrobenzoate | 2-Cl | 4-$CF_3$ | H | $NO_2$ | H | H | $-CH=CH-$ |
| 4-Hydroxy-2-buten-1-yl 5-(2,4-dichlorophenoxy)-2-nitrobenzoate | 2-Cl | 4-Cl | H | $NO_2$ | H | H | $-CH=CH-$ |
| 4-Hydroxy-2-butyn-1-yl 5-(2,4-dichlorophenoxy)-2-nitrobenzoate | 2-Cl | 4-Cl | H | $NO_2$ | H | H | $-C\equiv C-$ |
| 4-Hydroxy-2-butyn-1-yl 5-(2-chloro-4-trifluoromethylphenoxy)-2-nitrobenzoate | 2-Cl | 4-$CF_3$ | H | $NO_2$ | H | H | $-C\equiv C-$ |
| 2-Ethenyl-4-hydroxybutyl 5-(2-chloro-4-trifluoromethylphenoxy)-2-nitrobenzoate | 2-Cl | 4-$CF_3$ | H | $NO_2$ | H | H | $-CH_2CH_2-$<br>$CH=CH_2$ |
| 5-Hydroxy-2-hexen-1-yl 5-(2-chloro-4-trifluoromethylphenoxy)-2-nitrobenzoate | 2-Cl | 4-$CF_3$ | H | $NO_2$ | H | $CH_3$ | $-CH=CHCH_2-$ |
| 6-Hydroxy-2,4-hexadien-1-yl 5-(2-chloro-4-trifluoromethylphenoxy)-2-nitrobenzoate | 2-Cl | 4-$CF_3$ | H | $NO_2$ | H | H | $-CH=CHCH=CH-$ |

HERBICIDES REPRESENTATIVE OF GROUP A-V

| Compound | L | M | N | Y | $R_1$ | $R_2$ | R |
|---|---|---|---|---|---|---|---|
| 5-Hydroxy-3-hexen-2-yl 5-(2-chloro-4-trifluoromethylphenoxy)-2-nitrobenzoate | 2-Cl | $4-CF_3$ | H | $NO_2$ | $CH_3$ | $CH_3$ | $-CH=CH-$ |
| 2-(2-Hydroxyethyl)-4-pentyn-1-yl 5-(2-chloro-4-trifluoromethyl-phenoxy)-2-nitrobenzoate | 2-Cl | $4-CF_3$ | H | $NO_2$ | H | H | $-CHCH_2C\equiv CH$<br>$CH_2-$ |
| 4-Hydroxy-2-buten-1-yl 5-(2,4,6-trichlorophenoxy)-2-nitrobenzoate | 2-Cl | 4-Cl | 6-Cl | $NO_2$ | H | H | $-CH=CH-$ |
| 4-Hydroxy-2-buten-1-yl 5-(2-chloro-4-cyanophenoxy)-2-nitrobenzoate | 2-Cl | 4-CN | H | $NO_2$ | H | H | $-CH=CH-$ |
| 4-Hydroxy-2-buten-1-yl 5-(2,6-dinitrophenoxy)-2-nitrobenzoate | $2-NO_2$ | H | $6-NO_2$ | $NO_2$ | H | H | $-CH=CH-$ |
| 4-Hydroxy-2-buten-1-yl 5-(2-chloro-4-methoxyphenoxy)-2-nitrobenzoate | 2-Cl | $4-OCH_3$ | H | $NO_2$ | H | H | $-CH=CH-$ |
| 4-Hydroxy-2-butyn-1-yl 5-(2,4-dimethylphenoxy)-2-nitrobenzoate | $2-CH_3$ | $4-CH_3$ | H | $NO_2$ | H | H | $-C\equiv C-$ |

<u>HERBICIDES REPRESENTATIVE OF GROUP A-VI</u>

| Compound | L | M | N | X | X' | Y | $R_5$ | $R_8$ |
|---|---|---|---|---|---|---|---|---|
| [2-(2-Chlorophenoxyacetoxy) ethyl] 5-(2-chloro-4-trifluoromethylphenoxy)-2-nitrobenzoate | 2-Cl | 4-$CF_3$ | H | O | O | $NO_2$ | $-CH_2-CH_2-$ | 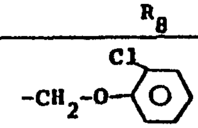 |
| {2-[2-(Ethoxyethoxycarbonyl)propionyloxy] ethyl} 5-(2-chloro-4-trifluoromethylphenoxy)-2-nitrobenzoate | 2-Cl | 4-$CF_3$ | H | O | O | $NO_2$ | $-CH_2CH_2-$ | $-CH_2CH_2-\overset{O}{\overset{\|}{C}}-OCH_2CH_2OCH_2CH_3$ |
| [8-(2,4-Dichlorophenoxyacetoxy)-3,5-dioxaoctyl] 5-(2-chloro-4-trifluoromethylphenoxy)-2-nitrobenzoate | 2-Cl | 4-$CF_3$ | H | O | O | $NO_2$ | $-(CH_2CH_2O)_2-CH_2CH_2-$ | 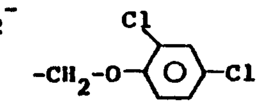 |
| [2-(2,4-Dichlorophenoxyacetoxy)ethyl] 5-(2-chloro-4-trifluoromethylphenoxy)-2-nitrobenzoate | 2-Cl | 4-$CF_3$ | H | O | O | $NO_2$ | $-CH_2CH_2-$ | 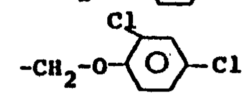 |
| [2-(Methyloxalyloxy) ethyl] 5-(2-chloro-4-trifluoromethylphenoxy)-2-nitrobenzoate | 2-Cl | 4-$CF_3$ | H | O | O | $NO_2$ | $-CH_2-CH_2-$ | $-\overset{O}{\overset{\|}{C}}-OCH_3$ |
| [2-(Ethyloxalyloxy)ethyl] 5-(2-chloro-4-(trifluoromethylphenoxy)-2-nitrobenzoate | 2-Cl | 4-$CF_3$ | H | O | O | $NO_2$ | $-CH_2-CH_2-$ | $-\overset{O}{\overset{\|}{C}}OCH_2CH_3$ |

## HERBICIDES REPRESENTATIVE OF GROUP A-VI

| Compound | L | M | N | X | X' | Y | $R_5$ | $R_8$ |
|---|---|---|---|---|---|---|---|---|
| { 2-[2-(Methoxycarbonyl) propionyloxy]ethyl } 5-(2-chloro-4-trifluoromethylphenoxy)-2-nitrobenzoate | 2-Cl | $4-CF_3$ | H | O | O | $NO_2$ | $-CH_2CH_2-$ | 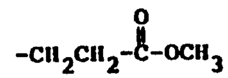 |
| [2-(Levulinyloxy)ethyl 5]-(2-chloro-4-trifluoromethylphenoxy)-2-nitrobenzoate | 2-Cl | $4-CF_3$ | H | O | O | $NO_2$ | $-CH_2CH_2-$ | 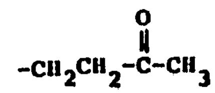 |
| { 2-[(2-Cyano-4-nitrophenoxy)-propionyloxy]ethyl} 5-(2-chloro-4-trifluoromethylphenoxy)-2-nitrobenzoate | 2-Cl | $4-CF_3$ | H | O | O | $NO_2$ | $-CH_2CH_2-$ | 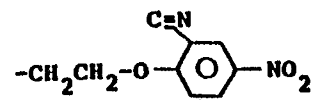 |
| [2-(2,4,6-Trichlorophenoxyacetamido)ethyl]5-(2-chloro-4-trifluoromethylphenoxy)-2-nitrobenzamide | 2-Cl | $4-CF_3$ | H | NH | NH | $NO_2$ | $-CH_2CH_2-$ | 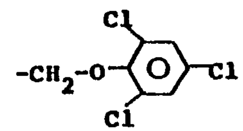 |
| [2-(Pyruvyl)ethyl]5-(2-chloro-4-trifluoromethylphenoxy)-2-nitrobenzoate | 2-Cl | $4-CF_3$ | H | O | O | $NO_2$ | $-CH_2CH_2-$ | 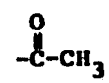 |

The herbicides of the present invention can be prepared by various methods. For example, the compounds defined in formula 1 where A represents group I are most advantageously prepared by reacting a substituted phenoxybenzoyl chloride with a suitable alcohol or thiol, or by direct esterification of the phenoxybenzoic acids with the alcohols or thiols according to the equation:

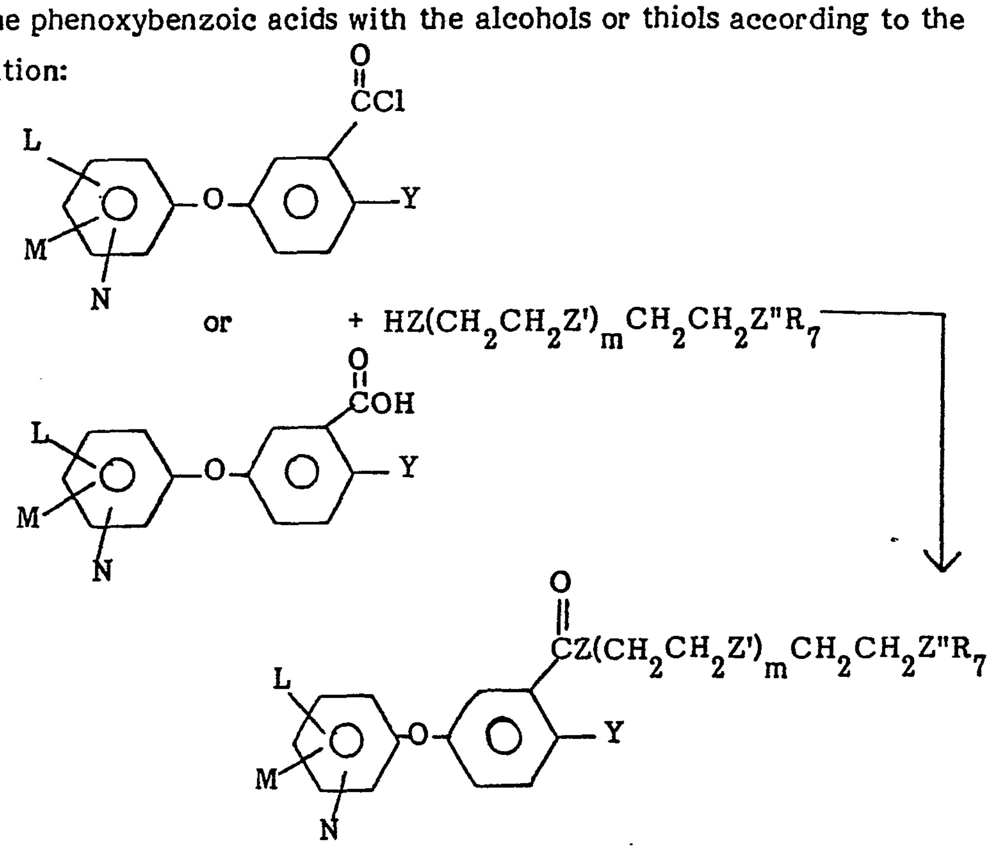


The nitrophenoxybenzoic acids are prepared either by reacting the sodium or potassium salt of 3-hydroxybenzoic acid with suitably substituted halobenzenes, followed by aromatic ring nitration with a conventional nitrating agent, or by reacting the sodium or potassium salt of a correspondingly substituted phenol with 5- halo-2-nitrobenzoic acid. The phenoxybenzoyl chlorides are synthesized by reacting the corresponding benzoic acid with a chlorinating agent such as thionyl chloride.

Those compounds represented by formula 1 wherein A is group II or group VI are advantageously prepared by reacting precurser (I) with (II); or precurser (III) with (IV), as follows:

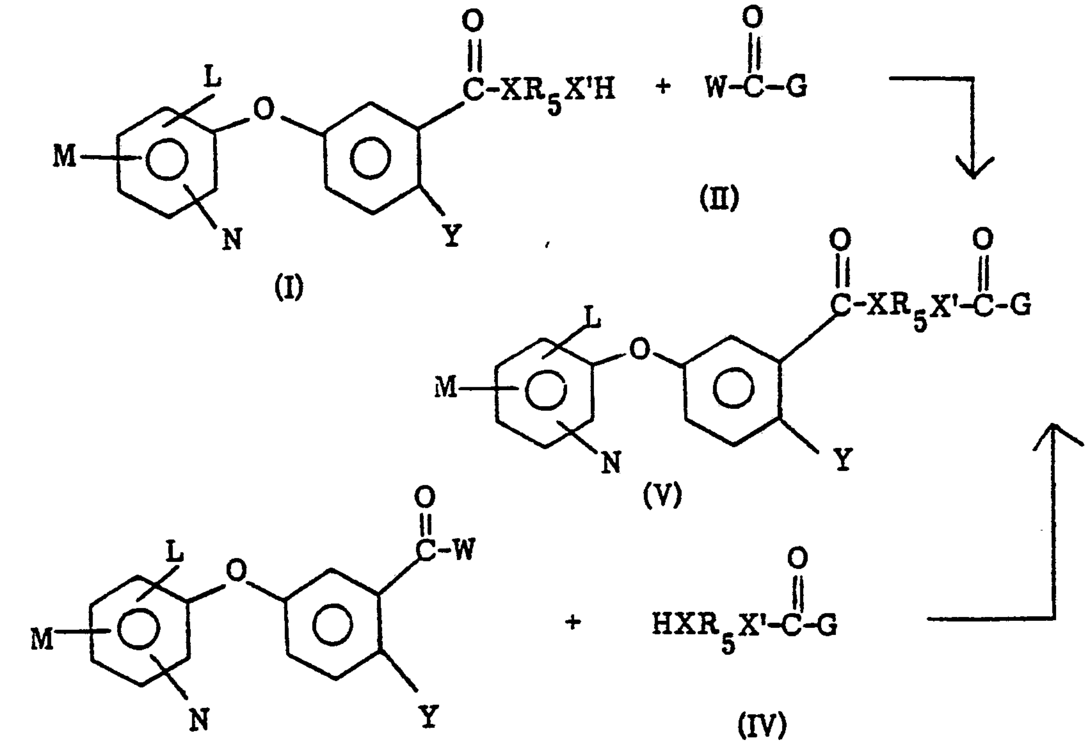

(I) (II) (V) (III) (IV)

where W in (II) and (III) is chlorine or bromine; G is $(X'')_nR_6$ or $R_8$; and L, M, N, Y, X, $R_5$, X', $(X'')_n$, $R_6$ and $R_8$ are as defined previously. The reactions may be carried out in the presence or absence of a base, and with or without a solvent.

Compound (I) in the equations is prepared by reacting m-phenoxybenzoic acid halide (VI) with reactant (VIII). Compound (IV) in the equations is synthesized by reacting an acid halide, 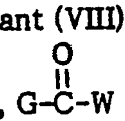 (VII) with reactant (VIII), as shown below.

These reactions

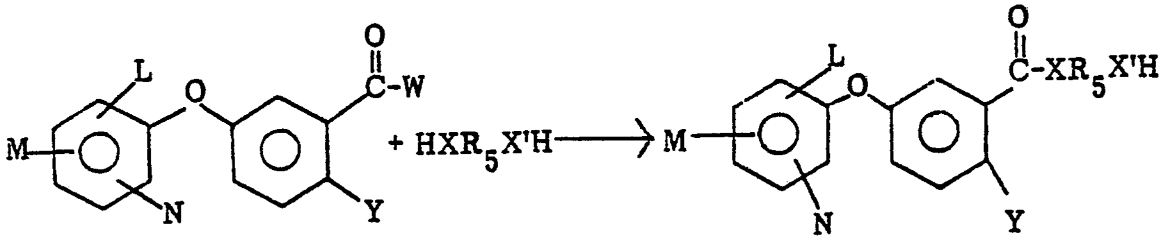

(VI) (VIII) (I)

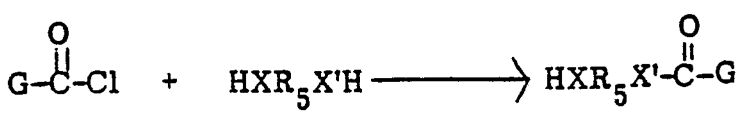

(VII) (VIII) (IV)

may be carried out in the presence or absence of a base, and with or without a solvent.

The X'H or XH functionality in (VIII) may be protected before condensation by reaction with a suitable protecting agent, such as chlorotrimethylsilane, which is removed later to regenerate the X'H or XH group.

The ester (I, X=X'=oxygen), is also prepared from the corresponding alcohols by reaction with m-phenoxybenzoic acid (XI) in the presence of a catalyst, preferably an acid as follows:

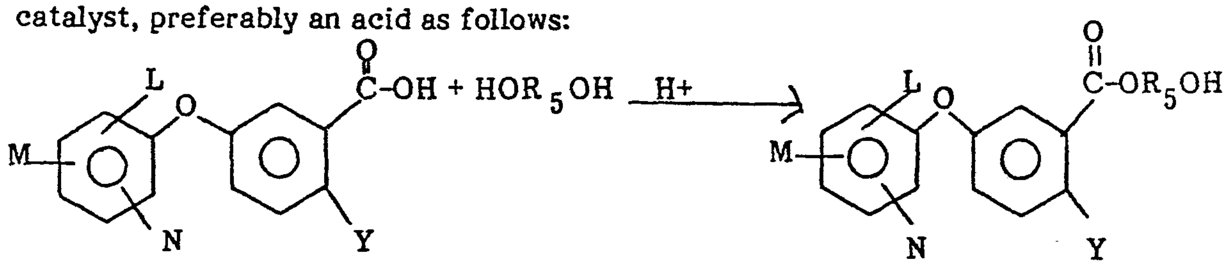


(IX) (I,X=X'=O)

Other methods of preparation will become apparent to those skilled in the art from the structure of the herbicides defined herein.

The herbicides represented in formula 1 having group A III are best prepared by reacting a correspondingly substituted phenoxybenzoyl chloride with a suitable polyhydroxy compound.

A convenient method for preparing the herbicides of formula 1 where A is group IV is illustrated by the reaction of a substituted phenoxybenzoyl chloride with a suitable alcohol, amine, or thiol, or by the direct acylation of the phenoxybenzoic acids with the alcohols, amines or thiols. Preferably the reaction is run at about a 2:1 ratio of, e.g. acid to alcohol. Alternatively, an excess of alcohol may be used, e.g. a 1:5 ratio of acid to alcohol, to produce the mono intermediate, followed by self-ester exchange at about $100^{\circ}$ - $250^{\circ}$C. while distilling out alcohol to produce the desired bis compound.

Finally, the herbicides of formula 1 wherein A represents group V are prepared by reacting a substituted phenoxybenzoyl chloride with a suitable diol or its alkali metal salt, or by direct esterification of the phenoxybenzoic acid with the diol.

All of the above synthesis are carried out at a temperature of between about $20^{\circ}$C. and $250^{\circ}$C. under 1-5 atmospheres, preferably under atmospheric pressure with agitation. Examples 1 through 70 in the following disclosure provide a more detailed description for the preparation of the above herbicidal compounds; although it is to be understood that other methods of synthesis may be employed if desired.

In addition to the above described processes, a novel and convenient method for preparing herbicides of Formula 1 having the side chains of A I, III, IV and V and also having a side chain where A is -X-alkylene-XH is described by the reaction of a phenoxybenzoic acid with a diacylating agent, such as a diol or dithiol to produce the corresponding bis compound.

Examples of suitable acylating agents include ethylene glycol, 2-hydroxyethyl ether, 2,2'-thiodiethanol, 1,2-ethanedithiol, ethylenediamine, 2-mercaptoethanol, 1,4-butenediol, 2-hydroxyethyl sulfide, and the like.

This novel synthesis is defined by a 2-, 3- or 4- step process wherein the first 2 steps involve reacting a substituted phenoxybenzoic acid having the formula

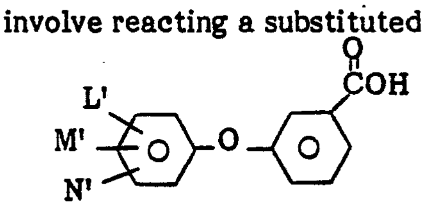

A.

wherein L', M', and N' are independently hydrogen, halogen, trihalomethyl, nitro, cyano, $C_{1-4}$ alkyl, $C_{1-4}$ alkoxy or $N<^{R_3}_{R_4}$ with a diacylating agent having the formula HXA'X'H wherein A' is $R_5$, $-\underset{R_1}{CH}-(R)_n-\underset{R_2}{CH}-$ or $-(CH_2)_nR_6(OH)_{p-1}$ at a temperature between about 20° and about 200°C. under from about 1 to about 5 atmospheres pressure for a period of from about 1 to about 40 hours; preferably at a temperature between about 100° and 150°C. under atmospheric pressure for a period of from about 10 to about 40 hours, to produce the corresponding bis-compound having the formula

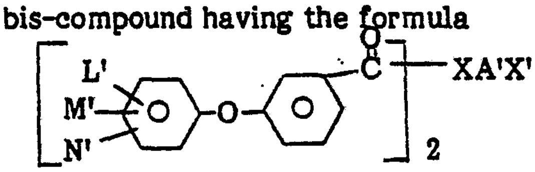

B.

and then nitrating the bis-compound with a conventional nitrating agent at a temperature between about -5° and about +70°C. under atmospheric pressure for a period of from about 1 to about 10 hours; preferably at a temperature between about 0°C. and about 30°C. for a period of from about 1 to about 5 hours, to produce the corresponding nitrated compound having the formula:

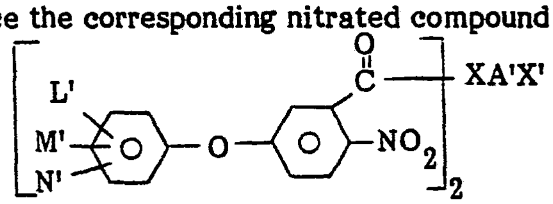

C.

It will be appreciated that when A' is $R_5$, the product of formula 1 A-IV is directly produced by the second step, i.e. nitration step C.

The above nitration step can be carried out in the absence or in the presence of an inert solvent, such as methylene dichloride, ethylene dichloride, chloroform, carbon tetrachloride, etc. Suitable nitrating agents include nitric acid/sulfuric acid, potassium nitrate/sulfuric acid, nitric acid/sulfuric acid/acetic anhydride and other conventional nitrating mixtures. When a solvent is employed, the concentration of the bis compound can be varied over a wide range limited only by economic considerations; however, it is found that concentrations between about10 and about 80 weight percent provides good results.

The mole ratio of diacylating agent to the phenoxybenzoic acid is most preferably about 1:2. The mole ratio of $NO_2^+$ in the nitrating agent is most preferably 2:1 with respect to the intermediate bis-compound; although a greater excess of nitrating agent, can be employed if desired.

When compounds of formula 1 having A group I, III and V are desired, the nitrated product of the 2 step process described above is transacylated by reacting it with at least an equimolar amount of an above-described diacylating agent at a temperature of between about 20° and about 250°C. under from about 1 to about 5 atmospheres pressure for a period of 0.5 to about 20 hours; preferably at a temperature between about 140° and about 200°C. under atmospheric pressure for a period of from about 1 to 10 hours. The reaction is generally defined by the equation:

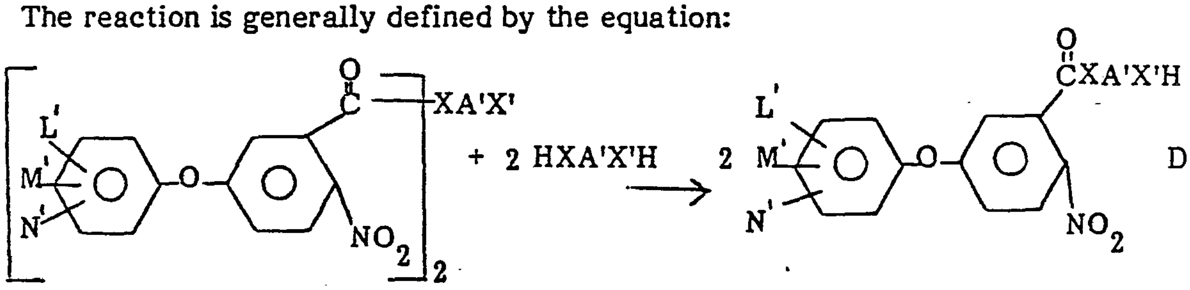

D.

The transacylating step is preferably carried out in a presence of a transacylating catalyst such as sulfuric acid, p-toluene sulfonic acid, hydrogen halide, hydrated manganese acetate, boron trifluoride etherate, phosphoric acid, calcium oxide, barium oxide, lead oxide, sodium oxide, lead nitrate, zinc acetate, manganese borate, zinc borate or an acidic ion exchange resin. Of these transesterification catalysts, manganese acetate tetrahydrate has been

found to be very effective. The mole ratio of diacylating agent employed in this third step of the reaction is between about 1:1 and 50:1, preferably between about 3:1 and about 20:1 with respect to the nitrated product of Step C. The excess and unreacted diacylating agent in this stage functions as a solvent for the transacylation reaction and is preferably separated from the product for recycle.

When the 3-step process is carried out in batch operation the various coreactants in each step are added sequentially after substantial completion of the preceding reaction. However, the synthesis may also be effected in separate reactors operated in series with heating and/or cooling devices between the various stages, as required. Also unreacted components may be separated from product and recycled to the appropriate stage.

To obtain the products of A groups II and VI, the product of equation D is reacted with a carbonyl-containing compound of the formula $HX-\underset{\underset{O}{\|}}{C}-G$

or the acid halide or anhydride of such carbonyl-containing compounds wherein X, X" and n are as defined above and G is $(X")_nR_6$ or $R_8$, according to the reaction defined by equation E;

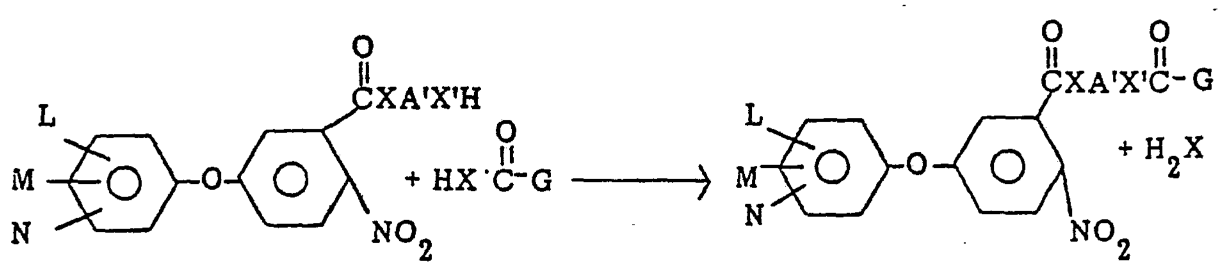

E.

Reaction E is effected at a temperature of between about 0°C. and about 200°C. under a pressure of from atmospheric to about 5 atmospheres over a period of from 0.5 to 20 hours; preferably at between about 25°C. and about 75°C. under atmospheric pressure over a period of from about 1 to 10 hours.

Suitable carbonylating agents include acetic acid, chloroacetic acid, thioacetic acid, N,N-dimethylcarbamyl chloride, pyruvic acid, propionic acid, methoxyacetyl chloride, acetic anhydride, phthalic anhydride, and the like.

The carbonyl-containing reactant is employed in a mole ratio of from about 1:1 to about 20:1 with respect to the product of reaction D. If desired the carbonylation can be effected in the presence of an inert solvent, e.g. tetrahydrofuran, and any of those previously given for the nitration reaction C. However, this reaction can be effected in the absence of solvent or in the presence of excess carbonyl-containing compound which excess may function as a recyclable solvent to this stage of the reaction.

The phenoxybenzoates of this invention may be applied in any amount which will give the required control of weeds depending on the type of weed and degree of infestation. A preferred rate of application of the benzoates is from 0.05 to 8 lbs. per acre. In practical application, the compounds may be applied in solid, liquid or in vaporized form, or, as it is generally done, as an active ingredient in a standard herbicidal composition or formulation which comprises a carrier. A generally accepted carrier is a substance which can be used to dissolve, disperse or diffuse the herbicidal components in the composition. Non-limiting examples of liquid carriers include water, organic solvents such as alcohols, ketones, halogenated hydrocarbons, aromatic hydrocarbons, ethers, amides, esters, nitriles, mineral oils, palm oil and the like. Non-limiting examples of solid carriers include Kaolin, bentonite, talc, diatomaceous earth, vermiculite, clay, gypsum, grain and seed hulls, ground corn cobs and the like. In addition to a carrier, it is usually desirable to add to the herbicidal formulation additives such as emulsifying agents, wetting agents, binding agents, stabilizer and the like or thickeners as desired for particular weather conditions or applications. The compounds may be formulated, for example, as a dust, wettable powders, paste, emulsifiable concentrates, granular formulations or as liquids or aerosols.

The phenoxybenzoates of this invention may be applied along with plant growth regulators, insecticides, fungicides, nematocides and fertilizers. They may be applied in combination with one or more other herbicides. Non-limiting examples of other herbicides which can be incorporated with the phenoxybenzoates of this invention are anilides, such as N-methoxymethyl (2,6-diethylphenyl) chloroacetamide; dinitroanilines, such as $\alpha,\alpha,\alpha$-trifluoro-2,6-dinitro-N,N-di-propyl-p-toluidine; carboxylic acids and derivatives; triazines; substituted ureas; carbamates; thiocarbamates; uracils; heterocycles and organo phosphorous compounds.

Reference is now had to the following examples which illustrate preferred embodiments of the invention set forth herein. It is to be understood, however, that these examples should not be construed as limiting to the scope of the invention as more broadly defined herein above and in the following claims.

EXAMPLE 1

Preparation of 6-Hydroxy-3,4-dithiahexyl 5-(2-chloro-4-trifluoromethylphenoxy)-2-nitrobenzoate

(A) 5-(2-chloro-4-trifluoromethylphenoxy)-2-nitro benzoyl chloride.

A solution of 5-(2-chloro-4-trifluoromethylphenoxy)-2-nitrobenzoic acid (448.1 g, 1.24 mole), thionyl chloride (458 g) and toluene (250 ml) was held at reflux for 8 hrs. The excess thionyl chloride and the solvent were stripped off under reduced pressure to give a reddish solid, which upon recrystallization from hexane-toluene afforded 282.9 g of the desired benzoyl chloride as a light yellow crystalline solid; mp 63-69$^{o}$C.

(B) 2-Hydroxyethyl 5-(2-chloro-4-trifluoromethylphenoxy)-2-nitrobenzoate.

A well-stirred solution of 5-(2-chloro-4-trifluoromethylphenoxy) benzoyl chloride (282.9 g, 0.74 mole) and ethylene glycol (1000 ml) was heated at 145$^{o}$C. for 3 hours. Then triethylamine (30 ml) was added. The solution was reheated at 142$^{o}$C. for 8 hours. After most of the ethylene glycol was distilled off under reduced pressure, the oil was taken up in 1700 ml of methylene chloride. The methylene chloride solution was washed three times with water, dried over $MgSO_4$ and concentrated to a gummy material. Molecular distillation afforded 226.2 g (75% yield) of a pale yellow gum which solidified on standing. 3.5 g of the solid was recrystallized from hexane-toluene to give 2.8 g of white solid; mp 75-78$^{o}$C.; nmr ($CDCl_3$) $\delta$ 0.3.32 (S, 1H), 3.67-4.12 (m, 2H), 4.15-4.63 (m, 2H), 7.02-8.25 (m, 6H); ir ($CHCl_3$) 1749 $Cm^{-1}$.

(C) 5-(2-Chloro-4-trifluoromethylphenoxy)-2-nitrobenzoyl chloride (15 g, 0.04 mole) was added to a solution of 2-hydroxyethyl disulfide (10 g, 0.065 mole) and triethylamine (10 ml). The resulting mixture was held at 100$^{o}$C. for 1 hr., cooled and was taken into 300 ml of ether. The ethereal solution was

washed two times with water, dried over Mg $SO_4$ and concentrated to 12.1 g of thick oil. The oil was column chromatographed through silica gel with 40% ethyl acetate - 60% hexane as eluent to yield 7.7 g of gum as pure 6-hydroxy-3,4-dithiahexyl 5-(2-chloro-4-trifluoromethylphenoxy)-2-nitrobenzoate; nmr ($CDCl_3$) $\delta$ 2.66 (S, 1H), 2.78-3.22 (m, 4H), 3.87 (t, 2H), 4.60 (t, 2H), 6.98-8.22 (m, 6H); ir ($CHCl_3$) 3630, 1752 $Cm^{-1}$.

## EXAMPLES 2 AND 3

The following compounds within the scope of this invention were prepared using procedures similar to that described in Example 1.

(2) 5-Hydroxy-3-thiapentyl{5-[2-chloro-4-(trifluoromethyl) phenoxy]-2-nitro}benzoate; nmr ($CDCl_3$) $\delta$ 2.96 (q, 4 H), 3.82 (t, 2 H), 4.54 (m, 2 H), 7.65-8.32 (m, 6 H), ir ($CHCL_3$) 3640 $Cm^{-1}$, 1743 $Cm^{-1}$.

(3) 6-Hydroxy-3,4-dithiahexyl [5-(2,4-dichlorophenoxy)-2-nitro]benzoate; nmr ($CDCl_3$) $\delta$ 2.40 (S, 1 H), 2.89 (t, 2 H), 3.04 (t, 2 H), 3.92 (t, 2 H), 3.63 (t, 2 H); 4.61 (t, 2 H), 6.92-8.22 (m, 6 H); ir ($CHCl_3$), 3630, 1750 $Cm^{-1}$.

EXAMPLE 4

Preparation of [2-Chloroacetoxyethyl] 5-(2-chloro-4-trifluoromethylphenoxy)-2-nitrobenzoate

Chloroacetyl chloride (3 g, 0.027 mole) was added dropwise to a stirred solution of (2-hydroxyethyl) 5-(2-chloro-4-trifluoromethylphenoxy)-2-nitrobenzoate (8.1 g, 0.02 mole), triethylamine (2.5 g), and ether (150 ml). The heat given off during the reaction raised the temperature of the ether solution to reflux. After completion of addition, the mixture was kept at reflux for 10 min. The mixture then was allowed to cool to room temperature, poured into 200 ml of water, and 400 ml of ether was added. The ether extract was washed three times with water, dried $CaSO_4$ and concentrated to 8.6 g of yellowish oil. The oil was column chromatographed through silica gel with 20% ethyl acetate-80% hexane as eluent to afford 3.2 g of pure (2-chloroacetoxyethyl) 5-(2-chloro-4-trifluoromethylphenoxy)-2-nitrobenzoate, as a pale yellow gumming material; nmr ($CDCl_3$) $\delta$ 4.16 (S, 2H), 4.50 (S, 4H), 6.98-8.18 (m, 6H); ir ($CHCl_3$) 1752, 1765.

EXAMPLES 5 THROUGH 25

The following compounds within the scope of this invention were prepared using procedures similar to that described in Example 4.

(5) (2-Acetoxyethyl) 5-(2-chloro-4-trifluoromethylphenoxy)-2-nitrobenzoate; nmr ($CDCl_3$) $\delta$ 2.06 (S, 3H), 4.47 (m, 4H), 7.02-7.89 (m, 6H); ir ($CHCl_3$) 1743, 1780 $Cm^{-1}$.

(6) [2-(Isobutyryloxy)ethyl] 5-(2-chloro-4-trifluoromethylphenoxy)-2-nitrobenzoate; nmr ($CDCl_3$) $\delta$ 1.13 (d, 6H), 2.58 (sept, 1H), 4.52 (m, 4H), 7.02-8.21 (m, 6H); ir ($CHCl_3$) 1743, 1750 $Cm^{-1}$.

( 7 ) [2-(Cyclopropanecarboxy)ethyl] 5-(2-chloro-4-trifluoromethyl phenoxy)-2-nitrobenzoate; ($CDCl_3$) $\delta$ 0.58-1.89 (m, 5H), 4.46 (m, 4H), 7.00-8.25 (m, 6H); ir ($CHCl_3$) 1740, 1749 $Cm^{-1}$.

( 8 ) [2-(Acryloyloxy)ethyl] 5-(2-chloro-4-trifluoromethylphenoxy)-2-nitrobenzoate; nmr ($CDCl_3$) $\delta$ 4.54 (m, 4H), 4.72-6.77 (m, 3H), 7.05-8.24 (m, 6H); ir ($CHCl_3$) 1747, 1753 $Cm^{-1}$.

(9 ) [2-(Methoxyacetoxy)ethyl] 5-(2-chloro-4-trifluoromethylphenoxy)-2-nitrobenzoate; nmr ($CDCl_3$) $\delta$ 3.42 (s, 3H), 4.06 (s, 2H), 4.50 (s, 4H), 6.79-8.23 (m, 6H); ir ($CHCl_3$) 1750 $Cm^{-1}$.

(10) [3-Acetoxypropyl] 5-(2-chloro-4-trifluoromethylphenoxy)-2-nitrobenzoate; nmr ($CDCl_3$) $\delta$ 2.17 (s, 3H), 2.00 (t, 2H), 2.60 (t, 2H), 4.35 (t, 2H), 7.03-8.23 (m, 6H); ir ($CHCl_3$) 1730, 1750 $Cm^{-1}$.

(11) [4-(Chloroacetoxy)butyl] 5-(2-chloro-4-trifluoromethylphenoxy)-2-nitrobenzoate; nmr ($CDCl_3$) $\delta$ 1.78 (t, 2H), 1.89 (t, 2H), 4.07 (s, 3H), 4.05-4.58 (m, 4H), 6.94-8.20 (m, 6H); ir ($CHCl_3$) 1747, 1733 $Cm^{-1}$.

(12) [2-(3-Chlorobenzoyloxy)ethyl] 5-(2-chloro-4-trifluoromethyl-phenoxy)-2-nitrobenzoate; nmr ($CDCl_3$) $\delta$ 4.66 (s, 4H), 6.98-8.24 (m, OH); ir ($CHCl_3$) 1738, 1750 $Cm^{-1}$.

(13) [2-(2,4-Dichlorobenzoyloxy)ethyl] 5-(2-chloro-4-trifluoromethyl phenoxy)-2-nitrobenzoate; nmr ($CDCl_3$) $\delta$ 4.66 (s, 4H), 6.70-8.62 (m, 9H); ir ($CH_3CN$) 1748 $Cm^{-1}$.

(14) [4-Acetoxy-2-buten-1-yl] 5-(2-chloro-4-trifluoromethylphenoxy)-2-nitrobenzoate; nmr ($CDCl_3$) $\delta$ 2.06 (s, 3H), 4.73 (d, 2H), 4.98 (d, 2H), 5.87 (t, 2H), 7.00-8.24 (m, 6H); ir ($CHCl_3$) 1743 $Cm^{-1}$.

(15) [11-Chloroacetoxy-3,6,9-trioxaundecyl] 5-(2-chloro-4-trifluoromethylphenoxy)-2-nitrobenzoate; nmr ($CDCl_3$) $\delta$ 3.32-4.62 (m, 18H), 7.92-8.20 (m, 6H); ir ($CHCl_3$) 1752, 1738 $Cm^{-1}$.

(16) [2-Benzoyloxyethyl] 5-(2-chloro-4-trifluoromethylphenoxy)-2-nitrobenzoate; nmr ($CDCl_3$) $\delta$ 4.66 (s, 4H), 6.96-8.30 (m, 11H); ir ($CHCl_3$) 1735, 1750 $Cm^{-1}$.

(17) [6-Acetoxy-3,4-dithiahexyl] 5-(2-chloro-4-trifluoromethylphenoxy)-2-nitrobenzoate; nmr ($CDCl_3$) $\delta$ 2.07 (s, 3H), 2.98 (q, 4H), 4.23 (t, 2H), 4.58 (t, 2H), 6.98-8.22 (m, 6H); ir ($CHCl_3$) 1748 $Cm^{-1}$.

(18) [2-(3-Amino-2,5-dichlorobenzoyloxy)ethyl] 5-(2-chloro-4-trifluoromethylphenoxy)2-nitrobenzoate; nmr (DMSO-$Cl_6$) $\delta$ 4.60 (s, 4H), 5.91 (s, 2H), 6.82-8.32 (m, 8H); ir (neat) 3495, 3400, 1742 $Cm^{-1}$.

(19) 2-(2-Chloropropionyloxy)ethyl 5-(2-chloro-4-trifluoromethylphenoxy)-2-nitrobenzoate; nmr ($CDCl_3$) $\delta$ 1.68 (d, 3H), 4.20-5.02 (m, 5H), 6.98-8.22 (m, 6H); 1745, 1755 $Cm^{-1}$.

(20) 2-(2-Methylbutyryloxy)ethyl 5-(2-chloro-4-trifluoromethylphenoxy)-2-nitrobenzoate; nmr ($CDCl_3$) $\delta$ 0.86 (t, 3H), 1.15 (d, 2H), 1.60 (m, 2H), 2.48 (h, 1H), 4.50 (m, 4H), 7.00-8.26 (m, 6H); ir ($CHCl_3$) 1750 $Cm^{-1}$.

(21) 2-(3,3-Dimethylacrylyloxy)ethyl 5-(2-chloro-4-trifluoromethylphenoxy)-2-nitrobenzoate; nmr ($CDCl_3$) $\delta$ 1.90 (d, 3H), 2.14 (d, 3H), 4.50 (m, 4H), 5.73 (m, 1H), 6.98-8.82 (m, 6H); ir ($CDCl_3$) 1725, 1745 $Cm^{-1}$.

(22) N-(2-Isobutyryloxy)ethyl-N-methyl 5-(2-chloro-4-trifluoromethylphenoxy)-2-nitrobenzoate, nmr ($CDCl_3$) $\delta$ 1.12 (d, 6H), 260 (m, 1H), 2.96 and 3.17 (2s, 3H), 3.40 and 3.80 (2t, 2H), 4.14 and 4.40 (2t, 2H), 6.72-8.48 (m, 6H); ir ($CHCl_3$) 1650, 1740 $Cm^{-1}$.

(23) N-(2-Acetoxyethyl)-N-isopropyl 5-(2-chloro-4-trifluoromethylphenoxy)-2-nitrobenzoate ($CDCl_3$) $\delta$ 1.10 (t, 6H), 2.02 (s, 3H), 3.22-3.89 (m, 3H), 4.38 (m, 2H), 6.74-8.36 (m, 6H); ir ($CHCl_3$) 1642, 1746 $Cm^{-1}$.

(24) N-(2-Acetoxyethyl) 5-(2-chloro-4-trifluoromethylphenoxy)-2-nitrobenzamide; nmr ($CDCl_3$) $\delta$ 2.00 (s, 3H), 3.62 (m, 2H), 3.18 (m, 2H), 6.62 (t, 1H), 6.82-8.22 (m, 6H); ir ($CHCL_3$) 3230, 1690, 1755 $Cm^{-1}$.

(25) N,N-[Di-(2-Acetoxy)ethyl] 5-(2-chloro-4-trifluoromethyphenoxy)-2-nitrobenzamide; nmr ($CDCl_3$) $\delta$ 2.03 (s, 6H), 3.46 (m, 2H), 3.83 (m, 2H), 4.20 (m, 2H), 4.44 (m, 2H), 6.82-8.42 (m, 6H); ir ($CHCl_3$) 1655, 1750 $Cm^{-1}$.

EXAMPLE 26

Preparation of [2-Propionyloxyethyl] 5-(2-chloro-4-trifluoromethylphenoxy)-2-nitrobenzoate

Propionyl chloride (2.9 g, 0.032 mole) was added dropwise to a solution of (2-hydroxy) 5-(2-chloro-4-trifluoromethylphenoxy)-2-nitrobenzoate (6 g, 0.016 mole) and triethylamine (25 ml). A yellowish salt was precipitated. The mixture then was heated to and held for 1.75 hr. at reflux, cooled and taken into 400 ml of methylene chloride. The methylene chloride solution was washed two times with water, dried over $MgSO_4$ and concentrated to an oil. Kugelrohr distillation at a temperature 170°C. afforded 5.7 g (91% yield) of colorless gum; nmr ($CDCl_3$) $\delta$ 1.09 (t, 3H), 2.37 (q, 2H), 4.45 (m, 4H), 7.00-8.22 (m, 6H); ir ($CHCl_3$) 1750 $Cm^{-1}$.

EXAMPLE 27

Preparation of [2-(Trifluoroacetoxy)ethyl] 5-(2-chloro-4-trifluoromethylphenoxy)-2-nitrobenzoate

Trifluoroacetic anhydride (8.4 g, 0.04 mole) was added portionwise to a solution of 2-hydroxyethyl 5-(2-chloro-4-trifluoromethylphenoxy)-2-nitrobenzoate (7g, 0.017 mole) and 50 ml of ether. After holding at 30-35°C. for an hour, the solution was allowed to stand at room temperature overnight. Excess trifluoroacetic anhydride was removed and 300 ml of ether was added. The ethereal solution was washed three times with water, dried over $CaSO_4$ and concentrated to an oil. Kugelrohr distillation afforded 3.6 g of pure 2-(trifluoroacetoxy)ethyl 5-(2-chloro-4-trifluoromethylphenoxy)-2-nitrobenzoate as a gumming material (yield : 42.2%); nmr ($CDCl_3$) $\delta$ 4.62 (s, 4H), 7.02-8.19 (m, 6H); ir ($CHCl_3$) 1752, 1803 $Cm^{-1}$.

EXAMPLE 28

Preparation of [5-(chloroacetoxy)-3-oxapentyl] 5-(2-chloro-4-trifluoromethylphenoxy)-2-nitrobenzoate

(A) [5-Hydroxy-3-oxapentyl] 5-(2-chloro-4-trifluoromethylphenoxy)-2-nitrobenzoate.

A mixture of 5-(2-chloro-4-trifluoromethylphenoxy)-2-nitrobenzoic acid (5.6 g, 0.015 mole), 2-hydroxyethyl ether (6.4 g, 0.06 mole) and p-toluenesulfonic acid (200 mg) was heated at 135-142°C. for 26 hrs. The resulting solution was cooled to room temperature, taken into 400 ml of ether. The ethereal solution was washed three times with water, dried over $CaSO_4$ and concentrated to 4.9 g of a yellowish gum. The gum was chromatographed on silica gel and eluted with hexane-ethyl acetate (90:10); yield 3.4 g of pure 5-hydroxy-3-oxapentyl 5-(2-chloro-4-trifluoromethylphenoxy)-2-nitrobenzoate as a pale yellow gum; nmr (DMSO-$d_6$) $\delta$ 3.56 (s, broad 5H), 3.76 (m, 2H), 4.52 (m, 2H), 7.18-8.40 (m, 6H); ir ($CHCl_3$) 3610, 1750 $Cm^{-1}$.

(B) 5-Hydroxy-3-oxapentyl 5-(2-chloro-4-trifluoromethylphenoxy)-2-nitrobenzoate (4 g, 0.009 mole) and triethylamine (2 ml) were dissolved in 200 ml of anhydrous ether in a 500 ml round-bottomed flask. Chloroacetyl chloride (5g, 0.44 mole) was added dropwise. The solution was held at reflux for 4.5 hrs. A large amount of salt precipitated. The ether solution was washed two times with sodium bicarbonate and three times of water, dried over $CaSO_4$ and concentrated to 3.6 g 5-(chloroacetoxy)3-oxapentyl 5-(2-chloro-4-trifluoromethylphenoxy)-2-nitrobenzoate as a gum; nmr ($CDCl_3$) $\delta$ 3.62-3.93 (m, 4H), 4.09 (s, 3H), 4.27-4.66 (m, 4H), 6.96-8.22 (m, 6H); ir ($CHCl_3$) 1754, 1768 $Cm^{-1}$.

EXAMPLE 29

Preparation of [6-(N,N-dimethylcarbamyloxy)hexyl] 5-(2-chloro-4-trifluoromethylphenoxy)-2-nitrobenzoate

(6-Hydroxyhexyl) 5-(2-chloro-4-trifluoromethylphenoxy)-2-nitrobenzoate (5 g, 0.014 mole) and N,N-dimethylcarbamyl chloride (11.68 g, 0.109 mole) were charged into a 80 ml round-bottomed flask. The solution was heated at 100°C. for 2 hrs, cooled and take into 300 ml of ether. The ethereal solution was washed three times with water, dried over $MgSO_4$. After the solvent being stripped off 5.9 g of [6-(N,N-dimethyl carbamyloxy)hexyl] 5-(2-chloro-4-trifluoromethylphenoxy)-2-nitrobenzoate was obtained as a brownish gum (yield : 86%); nmr ($CDCl_3$) $\delta$ 1.06-2.08 (m, 8H), 2.90 (s, 6H), 4.09 (t, 2H), 4.30 (t, 2H), 6.98-8.21 (m, 6H); ir ($CHCl_3$) 1696, 1742 $Cm^{-1}$.

EXAMPLES 30 THROUGH 36

The following compounds within the scope of this invention were prepared using procedures similar to that described in Example 29.

(30) [2-(N,N-Dimethylcarbamyloxy)ethyl] 5-(2-chloro-4-trifluoromethylphenoxy)-2-nitrobenzoate; nmr $\delta$ (DMSO-$d_6$) 2.86 (s, 6H), 4.20-4.76 (m, 4H), 7.22-8.29 (m, 6H); ir ($CHCl_3$) 1702, 1748 $Cm^{-1}$.

(31) [5-(N,N-Dimethylcarbamyloxy-3-oxapentyl] 5-(2-chloro-4-trifluoromethylphenoxy)-2-nitrobenzoate; nmr ($CDCl_3$) $\delta$ 2.92 (s, 6H), 3.72 (m, 4H), 4.20 (m, 2H), 4.50 (m, 2H), 7.00-8.26 (m, 6H); ir ($CHCl_3$) 1700, 1746 $Cm^{-1}$.

(32) [4-(N,N-Dimethylcarbamyloxy-2-buten-1-yl] 5-(2-chloro-4-trifluoromethylphenoxy)-2-nitrobenzoate; nmr ($CDCl_3$) $\delta$ 2.92 (s, 6H), 4.72 (m, 2H), 4.98 (m, 2H), 5.86 (m, 4H), 7.00-8.22 (m, 6H); ir ($CHCl_3$) 1750, 1710, 1594 $Cm^{-1}$.

(33) [4-(N,N-Dimethylcarbamyloxy)butyl] 5-(2,4-dichlorophenoxy)-2-nitrobenzoate; nmr ($CDCl_3$) $\delta$ 1.54-2.06 (m, 4H) 2.92 (s, 6H), 3.93-4.56 (m, 4H), 6.95-8.19 (m, 6H); ir ($CHCl_3$) 1702, 1748 $Cm^{-1}$.

(34) [2-(N,N-Diethylcarbamyloxy)ethyl] 5-(2-chloro-4-trifluoromethylphenoxy)-2-nitrobenzoate; nmr ($CDCl_3$) $\delta$ 110 (t, 6H), 3.28 (q, 4H), 4.49 (m, 4H), 6.98-8.23 (m, 6H); ir ($CHCl_3$) 1720, 1760 $Cm^{-1}$.

(35) {2-[N-(3-chlorophenyl)carbamyloxy]ethyl} 5-(2-chloro-4-trifluoromethylphenoxy)-2-nitrobenzoate; nmr (DMSO-$d_6$) 4.16-4.88 (m, 4H), 6.86-8.42 (m, 10H); ir ($CHCl_3$) 3245, 1604, 1750 $Cm^{-1}$.

(36) N-2-(N,N-Diethylcarbamyloxy)ethyl-N-propyl 5-(2-chloro-4-trifluoromethylphenoxy)-2-nitrobenzamide; nmr ($CDCl_3$) $\delta$ 1.12 (m, 9H), 3.25 (m, 6H), 3.54-4.60 (m, 4H), 6.82-8.65 (m, 6H); ir ($CHCl_3$) 1655, 1705 $Cm^{-1}$.

EXAMPLE 37

Preparation of 2-(N,N-Diallylcarbamylamino) ethyl 5-(2-chloro-4-trifluoromethylphenoxy) -2-nitrobenzoate

(A) N,N-Diallylcarbamyl chloride

In a 500 ml round-bottomed flask equipped with a condenser, mechanical stirrer, gas inlet, gas outlet and a trap 52.2 g of phosgene was dissolved in 300 ml of toluene. Diallylamine (51.3 g) was added dropwise over 1.5 hrs. The reaction mixture was then heated to and held at reflux for 1 hr., cooled and left overnight. The solvent was removed under reduced pressure. Pure 2-(N,N-diallylcarbamyl chloride (59.1 g, 70.1% yield) was collected at 58-60°C. (0.3 mm Hg), nmr (neat) 3.96 (S, broad, 4H), 4.95-6.20 (m, 6H).

(B) 2-(N,N-Diallylcarbamylamino)ethyl 5-(2-chloro-4-trifluoromethylphenoxy)-2-nitrobenzoate was prepared using procedures similar to that described in Example 37 (A).

EXAMPLE 38

Preparation of (3-Acetoxy-2,2-dimethylpropyl) 5-(2-chloro-4- trifluoromethylphenoxy)-2-nitrobenzoate

(A) (3-hydroxy-2,2-dimethyl-propyl) 5-(2-chloro-4-trifluoromethylphenoxy)-2-nitrobenzoate

In a three-necked 250 ml round-bottomed flask, sodium hydride (1.6 g, 60/40% in oil, 0.04 mole) was washed two times with hexane and pre-dried THF (100 ml) was added. 1,3-propandiol (27.4 g, 0.26 mole) then was added dropwise and the mixture was stirred for an hour at room temperature. {5-[2-chloro-4-(trifluoromethyl)phenoxy]-2-nitro} benzoyl chloride (10 g, 0.026 mole) was added in 1/2 hr. at a temperature below 30°C. The

mixture was heated to 57° for 1.25 hr., cooled, and concentrated under vacuum. The mixture then was taken up in 500 ml of ether, washed three times with water, dried over $MgSO_4$ and concentrated to an oil, Kugelrohr distillation of the oil afforded 5.35 g of the hydroxypropanyl ester. Purification of the oil through a silica gel column with 65% hexane — 35% ethyl acetate as an eluent gave 3.5 g of the pure (3-hydroxy-2,2-dimethyl-propyl) 5-(2-chloro-4- trifluoromethyl phenoxy)-2-nitro benzoate; nmr ($CDCl_3$) $\delta$ 0.96 (s, 6H), 2.48 (s, 1H), 3.40 (s, 2H), 4.15 (s, 2H), 7.05-8.22 (m, 6H); ir ($CHCl_3$) 3650, 1744 $Cm^{-1}$.

(B) Acetyl chloride (0.79 g, 0.01 mole) was added to a solution of (3-hydroxy-2,2-dimethyl-propyl) 5-(2-chloro-4-trifluoromethylphenoxy)-2-nitrobenzoate (0.25 g, 0.00056 mole) and THF (10 ml). The solution was held at reflux for 3 hrs., cooled and concentrated. The residue was taken into 250 ml of ether, washed two times with water and dried over $MgSO_4$. After the solvent was stripped off, 2.1 g of (3-Acetoxy-2,2-dimethylpropyl)-5-(2-chloro-4-trifluoromethylphenoxy)-2-nitrobenzoate was obtained as a yellowish gum, nmr ($CDCl_3$) $\delta$ 1.0 (s, 6H), 2.03 (s, 3H), 3.87 (s, 2H), 4.12 (s, 2H), 6.96-8.22 (m, 6H); ir ($CHCl_3$) 1743 $Cm^{-1}$.

EXAMPLE 39

Preparation of N-[2-(Acetoxy)ethyl]-N-methyl 5-(2-chloro-4-trifluoromethylphenoxy)-2-nitrobenzamide

(A) N-(2-Hydroxyethyl)-N-methyl 5-(2-chloro-4-trifluoromethylphenoxy)-2-nitrobenzamide.

5-(2-chloro-4-trifluoromethylphenoxy)-2-nitrobenzoyl chloride (7 g, 0.0184 mole) was added over 1 hr to a solution of N-methylaminoethanol (2.8 g, 0.0372 mole), triethylamine (3.7 g, 0.0368 mole) and THF (30 ml) at 25°-38°. The resulting mixture was heated to and held at reflux for 1.5 hrs., cooled and concentrated. The residue was taken into 400 ml of ether, washed three times with water, dried over $MgSO_4$ and concentrated to 7.8 g of crude product. Kugelrohr distillation under reduced pressure afforded 3.0 g of pure N-(2-Hydroxyethyl)-N-methyl 5-(2-chloro-4-trifluoromethylphenoxy)-2-nitrobenzamide as a gum; nmr ($CDCl_3$) $\delta$ 2.95-3.08 (2S, 3H); 3.22-3.82 (m) 4.74 (s, 1H), 7.04-8.52 (m, 6H); ir ($CHCl_3$) 3640, 3420, 1648 $Cm^{-1}$.

(B) Acetyl chloride (2.7 g, 0.0344 mole) was added dropwise in 5 mins. to a solution of N-(2-hydroxyethyl)-N-methyl 5-(2-chloro-4-trifluoromethylphenoxy)-2-nitrobenzamide (7.2 g, 0.0172 mole), triethylamine (25 ml) and THF (25 ml). The resulting mixture was heated up to and held at reflux for 2.5 hrs., cooled and concentrated. The residue was taken into 500 ml of ether, washed two times with water, dried over $MgSO_4$ and concentrated to 8.6 g of crude product. The crude product was purified on a column of silica gel using ethyl acetate cyclohexane (1:3) as eluent to yield 3.5 g of pure N-[2-(acetoxy)ethyl]-N-methyl 5-(2-chloro-4-trifluoromethylphenoxy)-2-nitrobenzamide as a gum; nmr ($CDCl_3$) $\delta$ 2.04 (s, 3H), 2.92 (s)-3.15 (s) (3H), 3.40 (t)-3.79 (t) (2H); 4.12 (t)-4.40 (t) (2H), 6.80-8.42 (m, 6H); ir ($CHCl_3$) 1750, 1655 $Cm^{-1}$.

EXAMPLE 40

Preparation of N-[2-(Acetylthio)ethyl] 5-(2-chloro-4-trifluoromethylphenoxy)-2-nitrobenzamide

(A) N-(2-Thioethyl) 5-(2-chloro-4-trifluoromethylphenoxy)-2-nitrobenzamide.

2-Aminoethanethiol hydrochloride (5.7 g, 0.050 mole) was placed in a 3-neck 100 ml round-bottom flask with rubber septum, magnetic stirrer, gas inlet and outlet valves. Acetonitrile (50 ml) and diisopropylethylamine (19.0 ml, 0.11 mole) were introduced under nitrogen. The resulting suspension was chilled to 0°C. and chlorotrimethylsilane (7.0 ml, 0.055 mole) was added at once with a 10 c.c. syringe. After 20 mins., 5-(2-chloro-4-trifluoromethylphenoxy)-2-nitrobenzoyl chloride (19.0 g, 0.050 mole) in 20 ml of acetonitrile was added dropwise over 0.5 hr. Diisopropylethylamine (8.7 ml, 0.050 mole) was added and the mixture was stirred at 0° for 1 hr. and at room temperature for 3 hrs. The mixture was poured into ice/water (150 ml), extracted with dichloromethane (2 x 75 ml). The extract was washed with water (2 x 50 ml), 5% hydrochloric acid (1 x 50 ml), 5% sodium bicarbonate (1 x 75 ml) and saturated sodium chloride solution (1 x 75 ml). After drying over sodium sulfate, the solvent was removed in vacuo to yield 19.5 g of N-(2-thioethyl) 5-(2-chloro-4-trifluoromethylphenoxy)-2-nitrobenzamide as a brown solid; m.p. (129-132°C.); nmr ($CDCl_3$)

δ 1.44 (t, 1H), 2.78 (q, 2H), 3.53 (q, 2H), 6.38-8.28 (m, 7H); ir ($CHCl_3$) 3452, 3320, 1675 $Cm^{-1}$.

(B) Acetyl chloride (2.1 ml, 0.038 mole) was added dropwise into a solution of N-(2-thioethyl) 5-(2-chloro-4-trifluoromethylphenoxy)-2-nitrobenzamide (5 g, 0.012 mole), triethylamine (5 ml) and acetonitrile (100 ml) at 0°C. The resulting mixture was stirred for 5 hrs. and poured into 300 ml of ether. The ether solution was washed three times with water, dried over $CaSO_4$ and concentrated to 4.7 g of orange gum. Kugelrohr distillation at 160°C. and 1.5 mm Hg afforded 3.5 g of pure N-[2-(acetylthio)ethyl]5-(2-chloro-4-trifluoromethylphenoxy)-2-nitrobenzamide; nmr ($CDCl_3$) δ 2.32 (s, 3H), 3.13 (m, 2H), 3.60 (m, 2H), 652 (s, broad, 1H), 6.89-8.20 (m, 6H); ir ($CHCl_3$) 3460, 3360, 1682, 1592 $Cm^{-1}$.

## EXAMPLE 41

### Preparation of[2-(N,N-Dimethylcarbamylthio)ethyl]5-(2-chloro-4-trifluoromethylphenoxy)-2-nitrobenzamide

N,N-Dimethylcarbamyl chloride (4 g, 0.037 mole) was added dropwise to a solution of N-(2-thioethyl) 5-(2-chloro-4-trifluoromethylphenoxy)-2-nitrobenzamide (5 g, 0.012 mole), triethylamine (5 ml) and acetonitrile (100 ml) at 0°C. After being stirred at 0°C. for 5.5 hrs. the solvent was stripped off under reduced pressure. The residue was taken into 400 ml of ether, washed three times with water, dried over $CaSO_4$ and concentrated to 6.2 g of yellow solid. The crude solid was chromatographed through a silica gel column (ethyl acetate: cyclohexane, 3:7), to give 4.2 g of pure 2-(N,N-dimethylcarbamyl-

thio)ethyl 5-(2-chloro-4-trifluoromethylphenoxy)-2-nitrobenzamide as a yellowish white solid; mp 101-102°C., nmr ($CDCl_3$) 2.95 (s, 6H), 3.10 (m, 2H), 3.63 (m, 2H), 6.80-8.24 (m, 6H); ir ($CHCl_3$) 3450, 3320, 1675, 1650 $Cm^{-1}$.

EXAMPLE 42

Preparation of{2-[N-(N,N-Diethylcarbamyl)amino]ethyl} 5-(2-chloro-4-trifluoromethylphenoxy)-2-nitrobenzoate

(A) N,N-Diethyl-N'-hydroxyethyl urea.

N,N-Diethylcarbamyl chloride (8.2 g, 0.06 mole) in THF (15 ml) was added dropwise to a solution of aminoethanol (3.08 g, 0.05 mole), triethylamine (6.08 g, 0.06 mole) and THF (25 ml) at 0°C. The mixture was stirred at room temperature for 60 hrs., filtered and concentrated to 9.0 g of N,N-diethyl-N'-hydroxyethyl urea as a pale yellow oil; nmr ($CDCl_3$) $\delta$ 1.15 (t, 3H), 2.88-3.84 (m, 8H), 5.26 (s, broad, 1H), ir (neat) 3380, 1620 $Cm^{-1}$.

(B) 5-(2-chloro-4-trifluoromethylphenoxy)-2-nitrobenzoyl chloride (3.8 g, 0.010 mole) in 15 ml of THF was added dropwise to a solution of N,N-diethyl-N'-hydroxyethyl urea (2.5 g, 0.015 mole), triethylamine (1.2 g, 0.012 mole) and THF (25 ml) at 0°C. The resulting mixture was stirred at room temperature overnight, filtered and concentrated. The residue was taken into 75 ml of methylene chloride, washed three times with a saturated $NaHCO_3$ solution and one time with water. After being dried over $Na_2SO_4$ and concentrated, a brown oil (5.0 g) was afforded. The oil was column chromatographed through silica gel

with 50% ethyl acetate - 50% cyclohexane as eluent to give 3.3 g of 2-[N-(N,N-diethylcarbamyl)amino]ethyl 5-(2-chloro-4-trifluoromethylphenoxy)-2-nitrobenzoate as a brown solid; mp 89-90°C.; NMR ($CDCl_3$) $\delta$ 1.12 (t, 6H), 3.23 (q, 4H), 3.67 (t, 2H), 4.46 (t, 2H), 4.95 (s, broad 1H), 6.92-8.28 (m, 6H); ir ($CHCl_3$) 3485, 1750, 1680 $Cm^{-1}$.

EXAMPLE 43

Preparation of [2-(N-Acetylamino)ethyl]5-(2-chloro-4-trifluoromethylphenoxy)-2-nitrobenzoate

N-Acetylethanolamine (2.9 g, 0.021 mole, 75% by wt. in $H_2O$) and toluene (60 ml) were introduced in a 100 ml round-bottom flask equipped with a Dean-Stark apparatus. The mixture was held at reflux for 2 hrs. to remove water from the solution. An oil separated from the toluene solution upon cooling. THF (50 ml) was added. To the resulting solution were added 5-(2-chloro-4-trifluoromethylphenoxy)(-2-nitrobenzoyl chloride) (8 g, 0.021 mole) and triethylamine (1 ml). The mixture was held at reflux for 1.67 hr. and concentrated. The residue was taken into 450 ml of ether, washed two times with $H_2O$, dried over $MgSO_4$, filtered and concentrated to yield 8.0 g of gum. The gum was column chromatographed through silica gel with 20% ethyl acetate - 80% cyclohexane as eluent to give 6.4 g of 2-(N-acetylamino)ethyl 5-(2-chloro-4-trifluoromethylphenoxy)-2-nitrobenzoate as a brownish gum nmr ($CDCl_3$) $\delta$ 1.98 (s, 3H), 3.58 (m, 2H), 4.42 (m, 2H), 6.25 (s, broad 1H), 6.98-8.22 (m, 6H); ir ($CHCl_3$) 3464, 1752, 1680 $Cm^{-1}$.

EXAMPLE 44

Preparation of [2,2-Bis(hydroxymethyl)propyl] 5-(2-Chloro-4-Trifluoromethylphenoxy)-2-Nitrobenzoate

To a solution of 1,1,1-tris(hydroxymethyl)ethanol (10 g., 0.083 mole), triethylamine (7.26 g, 0.07 mole) and tetrahydrofuran (250 ml) is added 5-(2-chloro-4-trifluoromethylphenoxy)-2-nitrobenzoyl chloride (10 g, 0.026 mole).

The mixture is held at reflux for 4 hrs., the solvent is stripped off and the residue is taken in to 400 ml of ethyl ether. The etheneal solution then is washed three times with water, dried over $CaSO_4$ and concentrated to 10.6 g of an amber gum. The gum is chromatographed on silica gel and eluted with ethyl acetate-hexane (30:70); yield 5.6 g of pure 2,2-bis(hydroxymethyl)propyl 5-(2-chloro-4-trifluoromethylphenoxy)-2-nitrobenzoate as a yellow gum; nmr ($CDCl_3$) 0.86 (S, 3H), 2.66 (S, 2H), 3.58 (S, 4H), 4.34 (S, 2H), 6.88-8.12 (m, 6H); ir ($CHCl_3$) 3660, 3540, 1750 $cm^{-1}$.

## EXAMPLE 45

### Preparation of (2,3-Dihydroxypropyl) 5-(2-Chloro-4-Trifluoromethyl phenoxy)-2-Nitrobenzoate

Glycerol (31.5 g, 0.34 mole), triethylamine (3.63 g, 0.035 mole) and THF (50 ml) are heated in a 100 ml flask with agitation. Then 5-(2-chloro-4-trifluoromethylphenoxy)-2-nitrobenzoyl chloride (15 g, 0.0395 mole) is added and the mixture is heated to 60-67°C. for 2 hrs., cooled and concentrated. The residue is taken into 400 ml of ether, washed two times with water and dried over $MgSO_4$ to afford 14.3 g of a yellowish gum. The gum is column chromatographed on silica gel and eluted with ethyl acetate-hexane (10:90); yield 5.0 g pure 2,3-dihydroxypropyl 5-(2-chloro-4-trifluoromethylphenoxy)-2-nitrobenzoate as a pale yellow gum, nmr ($CDCl_3$) $\delta$ 3.16 (S, 2H), 3.68 (d, 2H), 3.95 (m, 1H), 4.34 (d, 2H), 6.96-8.21 (m, 6H); ir ($CHCl_3$) 3520, 1750 $cm^{-1}$.

## EXAMPLE 46

### Preparation of 1,5-Bis-[5-(2-Chloro-4-Trifluoromethylphenoxy)-2-Nitrobenzoyloxy]-3-Oxa-Pentane

A mixture of 5-(2-chloro-4-trifluoromethylphenoxy)-2-nitrobenzoic acid (5.6g, 0.015 mole), 2-hydroxyethyl ether (5.59g, 0.035 mole) and p-toluenesulfonic acid (100 mg) was heated at 133°C for 18 hrs. The resulting solution was transferred into a Kugelrohr apparatus and further heated at 180 - 214°C at 1 m.m.

for 3 hrs. The solid residue obtained was dissolved in 400 ml of ether, washed three times with water, dried over $CaSO_4$ and concentrated to give 4.9 g of black oil. The oil was chromatographed through silica gel; eluted with ethyl acetate: hexane (1:4) to afford 2.9 g of the desired compound as a pure yellow gum; nmr ($CDCl_3$) $\delta$ 3.83 (m, 4H), 4.49 (m, 4H), 6.92 - 8.26 (m, 12H); ir ($CHCl_3$) 1750 $cm^{-1}$.

EXAMPLE 47

Preparation of 1,6-Bis-[5-(2-Chloro-4-Trifluoromethylphenoxy)-2-Nitrobenzoyloxy]-3, 4-Dithiahexane

5-(2-chloro-4-trifluoromethylphenoxy)-2-nitrobenzoyl chloride (15g, 0.0394 mole) was added to a solution of 2-hydroxyethyl disulfide (3.0g, 0.0197 mole), triethylamine (10 ml) and THF (100 ml). The mixture was heated and held at reflux for 6 hrs. After the THF was stripped off, the residue was taken into 500 ml of ether. The ethereal solution was washed three times with water, dried over $CaSO_4$ and concentrated to 15.1g of a brown oil. The oil was column chromatographed on silica gel, elute with ethyl acetate: hexane (3:7) to give 9.3g of the desired compound as a pure light yellow gum; nmr ($CDCl_3$) $\delta$ 3.00 (t, 4H), 4.59 (t, 4H), 6.98 - 8.25 (m, 12H); ir ($CHCl_3$) 1754 $cm^{-1}$.

EXAMPLES 48 THROUGH 58

The following compounds within the scope of this invention were prepared using procedures similar to that described in Example 47.

(48) 1, 4-Bis-[5-(2-chloro-4-trifluoromethylphenoxy)-2-nitrobenzoyloxy]butane; mmr ($CDCl_3$) $\delta$ 1.82 (t, 4H), 4.40 (t, 4H), 6.86 - 8.26 (m, 12H); ir ($CHCl_3$) 1750 $cm^{-1}$.

(49) 1, 6-Bis-[5-(2-chloro-4- trifluoromethylphenoxy)-2-nitrobenzoyloxy]hexane; nmr ($CDCl_3$) $\delta$ 1.01 - 2.20 (m, 8H), 4.36 (5, 4H), 6.98 - 8.24 (m, 12H); ir ($CHCl_3$) 1740 $cm^{-1}$.

(50) 1, 11-Bis-[5-(2-chloro-4-trifluoromethylphenoxy)-2-nitrobenzoyloxy]-3, 6, 9-trioxaundecane; nmr ($CDCl_3$) $\delta$ 3.62 (s, 8H), 3.70 (m, 4H), 4.48 (m, 4H), 6.88 - 8.16 (m, 12H); ir ($CHCl_3$) 1743 $cm^{-1}$.

(51) 1, 2-Bis-[5-(2-chloro-4-trifluoromethylphenoxy)-2-nitrobenzoyloxy]ethane; nmr ($CDCl_3$) $\delta$ 4.00 (s, 4H), 6.85 - 8.24 (m, 12H), ir ($CHCl_3$) 1755 $cm^{-1}$.

(52) 1, 3-Bis-[5-(2-chloro-4-trifluoromethylphenoxy)-2-nitrobenzoyloxy]propane; nmr ($CDCl_3$) $\delta$ 2.14 (q, 2H), 4.40 (t, 4H), 6.85 - 8.18 (m, 12H); ir ($CHCl_3$) 1750 $cm^{-1}$.

(53) 1, 10-Bis-[5-(2-chloro-4-trifluoromethylphenoxy)-2-nitrobenzoyloxy]decane, nmr ($CDCl_3$) $\delta$ 1.08 - 2.06 (m, 16H), 4.31 (t, 4H), 6.93 - 8.19 (m, 12H); ir ($CHCl_3$) 1748 $cm^{-1}$.

(54) 1, 5-Bis-[5-(2-chloro-4-trifluoromethylphenoxy)-2-nitrobenzoyloxy]-3-thiapentane; nmr ($CDCl_3$) $\delta$ 2.88 (t,4H), 4.48 (t, 4H), 6.96 - 8.18 (m, 12H); ir ($CHCl_3$) 1750 $cm^{-1}$.

(55) N-{2-[5-(2-chloro-4-trifluoromethylphenoxy)-2-nitrothiobenzoyloxy]}ethyl 5-(2-chloro-4-trifluoromethylphenoxy)-2-nitrobenzamide; nmr ($CDCl_3$) $\delta$ 3.40 (m, 2H), 3.68 (m, 2H), 6.70 (s, 1H), 6.88 - 8.24 (m, 12H); ir ($CHCl_3$) 3440, 1680, 1587 $cm^{-1}$.

(56) 1, 2-Bis-[5-(2-chloro-4-trifluoromethylphenoxy)-2-nitrothiobenzoyloxy]ethane, nmr ($CDCl_3$) $\delta$ 3.40 (s, 4H), 6.84 - 8.25 (m, 12H); ir ($CHCl_3$) 1692 $cm^{-1}$.

(57) 1, 4-Bis-[5-(2-chloro-4-trifluoromethylphenoxy)-2-nitrobenzoyloxy]-2-butene; nmr ($CDCl_3$) $\delta$ 4.99 (d, 4H), 5.92 (t, 2H), 6.95 - 8.22 (m, 12H); ir ($CHCl_3$) 1740 $cm^{-1}$.

(58) 1, 4-Bis-[5-(2-chloro-4-trifluoromethylphenoxy)-2-nitrobenzoyloxy]-2-butyne; nmr ($CDCl_3$) $\delta$ 5.02 (s, 4H), 6.93 - 8.26 (m, 12H).

EXAMPLE 59

Preparation of 1, 8-Bis- 5-(2-Chloro-4-Trifluoromethylphenoxy)-2-Nitrobenzoyl -3, 5-Dioxaoctane

A mixture of 5-(2-chloro-4-trifluoromethylphenoxy)-2-nitrobenzoic acid (10g, 0.028 mole), triethylene glycol (2.1g, 0.014 mole) and p-toluenesulfonic acid (300 mg) was stirred and heated at 140°c for 23 hrs. The reddish brown gum obtained was dissolved in 300 ml of ether, washed three times with water, dried over $CaSO_4$ and concentrated to give 10.8g of the desired compound as a yellowish gum; nmr ($CDCl_3$)$\delta$ 3.68 (s, 4H), 3.81 (m, 2H), 4.50 (m, 2H), 6.90 - 8.20 (m, 6H); ir ($CHCl_3$)1745 $cm^{-1}$.

EXAMPLE 60

Preparation of 2,4-bis[5-(2-chloro-4-trifluoromethylphenoxy)-2-nitrobenzoyloxy]hexane

Into a 200 ml of round-bottom flask were charged 5-(2-chloro-4-trifluoromethylphenoxy)-2-nitrobenzoic acid (8.0 g, 0.02 mole) 2,5-dibromohexane (2.5 g, 0.01 mole); KOH (4.4 g, 0.067 mole) and DMF 66 g. The mixture was heated at 105°C. for 24 hrs., cooled and taken into 600 ml of ether. The ethereal solution was washed with sodium bicarbonate solution and then with water. After being dried over $MgSO_4$, and concentrated, 6.8 g of clear yellow gummy material was yield. The crude gum was column chromatographed through silica gel with 10% ethyl acetate- % hexane as eluent to afford 2.0 g of pure 2,4-bis[5-(2-chloro-4-trifluoromethylphenoxy)-2-nitrobenzoyloxy]hexane as white solid; mp 102-105; nmr ($CDCl_3$) $\delta$ 1.35 (d, 6H), 1.74 (m, 4H), 5.16 (m, 2H), 6.88-8.27 (m, 12H); ir ($CHCL_3$) 1738 $Cm^{-1}$.

EXAMPLE 61

Preparation of (4-Hydroxy-2-Buten-1-yl) 5-(2-Chloro-4-trifluoromethylphenoxy)-2-Nitrobenzoate

5-(2-chloro-4-trifluoromethylphenoxy)-2-nitrobenzoic acid chloride (7.6 g, 0.02 mole) was added to a solution of 2-butene-1,4-diol (100 ml) and triethylamine (10 ml). The mixture was heated to 70°C. and held for 1.5 hr. Then substantially all of the excess 2-butene-1,4-diol was distilled off. The residue was taken into 700 ml of ether, washed three times with water, dried over $MgSO_4$ and concentrated to 8 g of crude 4-hydroxy-2-buten-1-yl 5-(2-chloro-4-trifluoromethylphenoxy)-2-nitrobenzoate. The crude gummy material was chromatographed on silica gel using 25% ethyl acetate-75% hexane as eluent to afford 3.4 g of pure (4-hydroxy-2-buten-1-yl) 5-(2-chloro-4-trifluoromethylphenoxy)-2-nitrobenzoate as a clear yellow gum; nmr ($CDCl_3$) $\delta$ 2.42 (S, 1H), 4.28 (d, 2H), 4.82 (d, 2H), 5.46-6.30 (m, 2H), 6.90-8.32 (m, 6H); ir ($CHCl_3$) 3625, 1742 $Cm^{-1}$.

EXAMPLES 62 AND 63

The following compounds of this invention were prepared using procedures similar to that described in Example 61.

(62) (4-Hydroxy-2-buten-1-yl) 5-(2,4-dichlorophenoxy)-2-nitro benzoate; nmr ($CDCl_3$) 4.16 (d, 2H), 4.92 (d, 2H), 5.82 (m, 2H), 6.98-8.44 (m, 6H); ir ($CHCl_3$), 3612, 1746 $Cm^{-1}$.

(63) (4-Hydroxy-2-butyn-1-yl) 5-(2,4-dichlorophenoxy)-2-nitrobenzoate; nmr ($CDCl_3$) $\delta$ 2.58 (S, 1H), 4.30 (t, 2H), 4.92 (t, 2H), 6.86-8.23 (m, 6H); ir ($CHCl_3$) 3624, 1756 $Cm^{-1}$.

EXAMPLE 64

Preparation of (4-Hydroxy-2-Butyn-1-yl) 5-(2-Chloro-4-trifluoromethylphenoxy)-2-Nitrobenzoate

In a three-necked 250 ml round-bottomed flask, sodium hydride (3.2 g, 60/40% in oil, 0.08 mole) was washed two times with hexane and pre-dried THF (150 ml) was added. 2-Butyn-1,4-diol (34 g, 0.4 mole) then was added dropwise and the mixture was stirred for an hour at room temperature. {5-[2-chloro-4-(trifluoromethyl)phenoxy]-2-nitro} benzoyl chloride (15 g, 0.04 mole) was added at a temperature below 35°C.

The mixture then was heated at reflux for 1 hr., cooled, and concentrated on rotovap. The mixture was taken up in 500 ml of ether. The ether solution was washed three times with water, dried over $MgSO_4$ and concentrated to an oil. 6.5 g of the oil was chromatographed on silica gel, using 20% ethylacetate - 80% hexane as eluent, to afford 3.2 g of pure 4-hydroxy-2-butyn-1-yl 5-(2-chloro-4-trifluoromethylphenoxy)-2-nitrobenzoate; nmr ($CDCl_3$) $\delta$ 2.62 (S, 1H), 4.27 (S, 2H), 4.94 (S, 2H), 6.98-8.26 (m, 6H); ir ($CHCl_3$) 3623, 1757 $Cm^{-1}$.

EXAMPLE 65

Preparation of [8-(2,4-dichlorophenoxyacetoxy)-3,6-dioxaoctyl] 5-(2-chloro-4-trifluoromethylphenoxy)-2-nitrobenzoate

A. 2,4-Dichlorophenoxyacetyl chloride

A mixture of 2,4-dichlorophenoxyacetic acid (38 g) and thionyl chloride (100 ml) was held at reflux for 2 hours. The excess thionyl chloride was removed from the resulting clear solutions under reduced pressure to yield 43.7 g pale yellow oil; ir ($CHCl_3$) 1800 $Cm^{-1}$; nmr ($CDCl_3$) $\delta$ 495 (S, 2H), 688-7.42 (m, 3H).

B. 8-Hydroxy-3,6-dioxaoctyl 2,4-dichlorophenoxyacetate

2,4-Dichlorophenoxyacetyl chloride (40.0 g, 0.18 mole) was added dropwise to a solution of trimethylene glycol (32 g, 0.21 mole) and triethylamine (21 g) over 1 hour. As the chloride was added a salt precipitated out and stirring became difficult. Another 10 ml of trimethylene glycol and 50 ml of THF was added. The mixture was held at reflux for 3 hours. After the solvent was stripped off, the residue was taken into 650 ml of ether, washed two times with $NaHCO_3$ solution and two times with water. The ether solution afforded 40.6 g of brownish gum. A sample of 20 g of the gum was run through a silica gel column with 10% to 60% ethyl acetate and 90% to 40% hexane as eluent to yield 5.3 g of pure 8-hydroxy-3,6-dioxaoctyl 2,4-dichlorophenoxyacetate as a yellowish oil, nmr ($CDCl_3$) $\delta$ 2.73 (S, 1H), 3.34 (m, 10H), 4.18-4.49 (m, 2H), 4.73 (S, 2H), 6.62-7.48 (m, 3H); ir ($CHCl_3$) 1750, 1770, 3500, 3620 $Cm^{-1}$.

C. (2-Hydroxyethyl) 5-(2-chloro-4-trifluoromethylphenoxy)-2-nitrobenzoyl chloride (4.0 g, 0.0105 mole) was added portionwise to a solution of 8-hydroxy-3,6-dioxaoctyl 2,4-dichlorophenoxyacetate (3.5 g, 0.103 mole), triethylamine (1.2 g, 0.103 mole) and THF (40 ml) over 5 minutes. The resulting solution was held at reflux for 3 hours. After the solvent was stripped off, the residue was taken into 400 ml of ether, washed two times with $NaHCO_3$ solution and two times with water. The ether solution was then dried over $MgSO_4$ and concentrated to 5.9 g of brownish gum; nmr ($CDCl_3$) $\delta$ 3.30-4.02 (m, 8H), 4.25-4.62 (m, 4H), 4.70 (S, 2H), 6.65-8.24 (m, 9H); ir ($CHCl_3$) 1750, 1770 $Cm^{-1}$.

## EXAMPLE 66

Preparation of [2-(2,4-Dichlorophenoxyacetoxy)ethyl] 5-(2-chloro-4-trifluoromethylphenoxy)-2-nitrobenzoate

2,4-Dichlorophenoxyacetyl chloride (5.3 g, 0.25 mole), was added portionwise to a solution of 2-hydroxyethyl 5-(2-chloro-4-trifluoromethylphenoxy)-2-nitrobenzoate (10 g, 0.25 mole) and pyridine (70 ml). The resulting mixture was heated to 60°C. for 1 hr., cooled and taken into 500 ml $CH_2Cl_2$. The methylene chloride solution was washed two times with water, dried and concentrated to 12.5 g of gummy material. The crude gum was chromatographed through silica gel (3:10; ethyl acetate:hexane) to give 2.3 g of pure [2-(2,4-dichlorophenoxyacetoxy)ethyl] 5-(2-chloro-4-trifluoromethylphenoxy)-2-nitrobenzoate; nmr (DMSO) 4.20-4.76 (m, 4H), 5.04 (S, 2H), 6.96-8.42 (m, 9H); ir ($CHCl_3$) 1755, 1770 $Cm^{-1}$.

## EXAMPLE 67

Preparation of [2-(Methyloxalyloxy)ethyl] 5-(2-chloro-4-trifluoromethylphenoxy)-2-nitrobenzoate

Methyloxalyl chloride (2.3 g, 0.0 188 mole) was added dropwise to a solution of (2-hydroxyethyl) 5-(2-chloro-4-trifluoromethylphenoxy)-2-nitrobenzoate (5 g, 0.123 mole), 20 ml of THF

(tetrahydrofuran) and triethylamine (1.5 g, 0.127 mole) in 10 minutes. The temperature of reaction solution rose from 23°C. to 40°C. The resulting light yellow solution with white precipitate was heated and held at reflux for 1 hour. The solvent was stripped off and the residue was taken into 400 ml of ether. The ethereal solution was washed two times with $NaHCO_3$ solution and two times with water, dried over $MgSO_4$ and concentrated to 5.7 g of the desired product as a clear yellow gum, nmr ($CDCl_3$)$\delta$ 3.88 (S, 3H), 4.60 (S, 4H), 6.96-8.22 (m, 6H); ir ($CHCl_3$) 1757, 1782 $Cm^{-1}$.

EXAMPLE 68

Preparation of [2-(ethyloxalyloxy)ethyl] 5-(2-chloro-4-trifluoromethylphenoxy)-2-nitrobenzoate

Ethyloxalyl chloride (2.3 g, 0.0 188 mole) was added dropwise to a solution of (2-hydroxyethyl) 5-(2-chloro-4-trifluoromethylphenoxy) -2-nitrobenzoate (5 g, 0.123 mole), 20 ml of THF and triethylamine (1.5 g, 0.127 mole) in 10 minutes. The temperature of reaction solution rose from 23°C. to 40°C. The resulting light yellow solution with white precipitate was heated and held at reflux for 1 hour. The solvent was stripped off and the residue was taken into 400 ml of ether. The ethereal solution was washed two times with $NaHCO_3$ solution and two times with water, dried over $MgSO_4$ and concentrated to 5.7 g of the desired product as a clear yellow gum, ($CDCl_3$) $\delta$ 1.37 (t, 3H), 4.40 (q, 2H), 4.63 (S, 1H), 6.98-8.28 (m, 6H); ir ($CHCl_3$) 1750, 1770 $Cm^{-1}$.

EXAMPLE 69

Preparation of {2-[2-(methoxycarbonyl)propionyloxy]ethyl} 5-(2-chloro-4-trifluoromethylphenoxy)-2-nitrobenzoate

Carbomethoxypropionyl chloride (2.8 g, 0.0186 mole) was added dropwise to a solution of (2-hydroxyethyl) 5-(2-chloro-4-trifluoromethylphenoxy)-2-nitrobenzoate (5 g, 0.0123 mole), THF (20 ml) and triethylamine (1.5 g, 0.0127 mole) over 30 minutes. The temperature rose from 23° to 45°C. and yellow solid precipitated out. The mixture

was then heated to reflux for 0.5 hours, cooled and concentrated under reduced pressure. The residue was taken into 400 ml of ether and 200 ml of saturated $NaHCO_3$ solution. After being washed two times with water, dried over $MgSO_4$, 6.3 g of clear yellow oil was obtained. The oil is the desired benzoate; nmr ($CDCl_3$) $\delta$ 2.63 (S, 4H), 3.68 (S, 3H), 4.27-4.72 (m, 4H), 6.96-8.20 (m, 6H); ir ($CHCl_3$) 1745 $Cm^{-1}$ (broad).

EXAMPLE 70

Preparation of 2-(Levulinylox)ethyl 5-(2-chloro-4-trifluoromethylphenoxy)-2-nitrobenzoate

This compound was prepared using procedures similar to that described in Example 4; nmr ($CDCl_3$) $\delta$ 2.20 (S, 3H), 2.68 (m, 4H), 4.42 (m, 4H), 6.92-8.28 (m, 8H); ir ($CHCl_3$) 1733, 1746 $Cm^{-1}$.

The present application discloses several methods for preparing the present herbicides, among which is a novel process comprising 2,3 or 4 Steps. Examples 71-73 illustrate this novel process.

EXAMPLE 71

Preparation of 1,2-Bis[5-(2-chloro-4-trifluoromethylphenoxy)-2-nitrobenzoyloxy]ethane

A. In a 250 ml glass flask, xylene (100 cc), 3-(2 -chloro-4 -trifluoromethylphenoxy) benzoic acid (131.6 g., 0.1 mole), ethylene glycol (6.2 g., 0.1 mole) and conc. sulfuric acid (2 g.) were heated at reflux (130°C.) while azeotroping off about 2 cc of a water-glycol mixture. The temperature was maintained for 30 hrs., after which the mixture was cooled to room temperature. The product mixture was then analyzed by thin layer chromatography (10:1 toluene/ethyl acetate on silica) and the following product separations were reported: $R_f$ = 0.71 (major spot - product), 0.47 (monoester), and 0.13 (starting material). The reaction mixture was partitioned between ethyl acetate and a 5% sodium bicarbonate solution. The organic phase was reduced in volume by evaporation leaving 25.4 g of crude bis-ester, which was subjected to column chromatography eluting with a 3:1 toluene/$CH_2Cl_2$ solvent

mixture. The product fractions averaged 20.6 g. (62.6%) upon evaporation. Recrystallization from 5:1 hexane/toluene yielded 16.2 g. of 1,2-bis [5-(2-chloro-4-trifluoromethylphenoxy)benzoyloxy]ethane; m.p. 98°C.; nmr ($CDCl_3$); $\delta$ 8.1-6.9 (7, m, CH-Ar), 4.7 (4, s, $CH_2$); ir (neat) 1726 $cm^{-1}$ (C=O).

B. A solution of 1,2-bis[5-(2-chloro-4-trifluoromethylphenoxy)benzoyloxy] ethane (6.6 g., 0.01 mole) in 15 cc of dichloroethane was treated with 10.4 g. of ca. 33/67 nitric/sulfuric acids at 2-4°C. for 1.75 hours. Thin layer chromatography (3:1 toluene/$CH_2Cl_2$ on silica) analysis indicated a product with $R_f$ = 0.39. Water (15 cc) was added dropwise at 0-5°C. The layers were separated and the organic phase was washed sequentially with 5% sodium bicarbonate and water. The solvent was removed by rotary evaporation leaving 6.3 g. of 1,2-bis[5-(2-chloro-4-trifluoromethylphenoxy)-2-nitrobenzoyloxy] ethane (84%); nmr ($CDCl_3$)$\delta$ 8.1-7.0 (10, m, CH-Ar), 4.6 (4, s, $CH_2$); ir (neat) 1750 (C=O), 1530 $cm^{-1}$ ($NO_2$).

Other compounds having the general formula 1 where A is group IV are prepared by the above procedure A. and B. except for substitution of the ethylene glycol reactant in step A.

Accordingly, 1,3-bis[5-(2-chloro-4-trifluoromethylphenoxy)-2-nitrobenzoyloxy]propane is prepared by substituting 1,3-propandiol for ethylene glycol in Step A; 1,5-bis[5-(2-chloro-4-trifluoromethylphenoxy)-2-nitrobenzoyloxy]-3-oxapentane is prepared by substituting 2-hydroxyethyl ether for ethylene glycol in Step A; and 1,6-bis [5-(2-chloro-4-trifluoromethylphenoxy)-2-nitrobenzoyloxy] 3,4-dithiahexane is prepared by substituting 2-hydroxyethyl disulfide for ethylene glycol in Step A.

EXAMPLE 72

Preparation of [2-Hydroxy ethyl] 5-(2-chloro-4-trifluoromethylphenoxy)-2-nitrobenzoate

The reaction product of Example 1 B, 1,2-bis [5-(2-chloro-4-trifluoromethylphenoxy)-2-nitrobenzoyloxy] ethane (6.1 g, 0.008 mole) was mixed with ethylene glycol (10.1 g., 0.16 mole) and manganese acetate tetrahydrate (0.4 g.) and heated at 165-185$^{o}$C. for 5 hours, and then cooled to room temperature. The product mixture was then subjected to thin layer chromatographic analysis (5:1 toluene/ethyl acetate on a silica gel support); which showed a single spot with $R_f$ = 0.2 identified as 2-hydroxyethyl 5-(2-chloro-4-trifluoromethylphenoxy)-2-nitrobenzoate. This indicated that complete transacylation had taken place.

The mixture was partitioned between water and toluene/ethyl acetate and the volume of the organic phase was reduced by rotary evaporation. The resulting product was then subjected to high pressure liquid chromatography purification employing a 5:1 toluene/ethyl acetate solvent system. The product fractions (4.2 g, 65%) were identified by Thin Layer Chromatography, nmr and ir. High pressure liquid chromatography analysis revealed a 88.3% purity with a trace of bis-nitro ester and less than 1% 5-[2-chloro-4-trifluoromethylphenoxy] -2-nitrobenzoic acid.

The reaction of this example can be employed to make other nitrobenzoates by proper substitution of 1,2-bis [5-(2-chloro-4-trifluoromethylphenoxy)-2-nitrobenzoyloxy] ethane. For example, [3-hydroxy propyl] 5-(2-chloro-4-trifluoromethylphenoxy)-2-nitrobenzoate is prepared by substituting 1,3-bis [5-(2-chloro-4-trifluoromethylphenoxy)-2-nitrobenzoyloxy] propane; [5-hydroxy-3-oxapentyl] 5-(2-chloro-4-trifluoromethylpehnoxy)-2-nitrobenzoate is prepared by substituting 1,5 bis [5-(2-chloro-4-trifluoromethylphenoxy)-2-nitrobenzoyloxy] -3-oxapentane; [5-hydroxy-3-thio-pentyl] 5-(2-chloro-4-trifluoromethylphenoxy)-2-nitrobenzoate is prepared by substituting 1,5-bis [5-(2-chloro-4-trifluoromethylphenoxy)-2-nitrobenzoyloxy] -3-thiapentane and [4-hydroxy-2-buten-1-yl] 5-(2-chloro-4-trifluoromethylphenoxy)-2-nitrobenzoate is prepared by substituting 1,4-bis [5-(2-chloro-4-trifluoromethylphenoxy)-2-nitrobenzoyloxy] -2-butene for 1,2 bis [5-(2-chloro-4-trifluoromethylphenoxy)-2-nitrobenzoyloxy] ethane in this example.

EXAMPLE 73

Preparation of [2-Chloroacetoxyethyl] 5-(2 chloro-4-trifluoromethylphenoxy)-2-nitrobenzoate

Chloroacetyl chloride (3 g, 0.027 mole) was added dropwise to a stirred solution of [2-hydroxyethyl] 5-(2-chloro-4-trifluoromethylphenoxy)-2-nitrobenzoate (8.1 g, 0.02 mole), triethylamine (2.5 g), and ether (150 ml). The heat given off during the reaction raised the temperature of the ether solution to reflux. After completion of addition, the mixture was kept at reflux for 10 min. The mixture then was allowed to cool to room temperature, poured into 200 ml of water, and 400 ml of ether was added. The ether extract was washed three times with water, dried $CaSO_4$ and condentrated to 8.6 g of yellowish oil. The oil was column chromatographed through silica gel with 20% ethyl acetate-80% hexane as eluent to afford 3.2 g of pure [2-chloroacetoxyethyl] 5-(2-chloro-4-trifluoromethylphenoxy)-2-nitrobenzoate, as a pale yellow gummy material; nmr ($CDCl_3$) $\delta$ 4.16 (S, 2H), 4.50 (S, 4H), 6.98-8.18 (m, 6H); ir ($CHCl_3$) 1752, 1765.

Other carbonylated compounds can be prepared by the procedure of this example by proper substitution of chloroacetyl chloride. Thus, [2-(methoxyacetoxy)ethyl] 5-(2-chloro-4-trifluoromethylphenoxy)-2-nitrobenzoate is prepared by substituting methoxyacetyl chloride for chloroacetyl chloride; [2-acetoxyethyl] 5-(2-chloro-4-trifluoromethylphenoxy)--nitrobenzoate is prepared by substituting acetic anhydride for chloroacetyl chloride; [2-(propionyloxy)ethyl] 5-(2-chloro-4-trifluoromethylphenoxy)-2-nitrobenzoate is prepared by substituting propionyl chloride for chloroacetyl chloride and [2-(N,N-dimethylcarbamyloxy) ethyl] 5-(2-chloro-4-trifluoromethylphenoxy)-2-nitrobenzoate is prepared by substituting N,N-dimethylcarbamyl chloride for chloroacetyl chloride.

## HERBICIDAL TESTS

Compounds from each of the A categories were tested for herbicidal activity and the results of these tests are reported as follows.

### EXAMPLE 74

Tests were made on two flats seeded with species of representative monocotyledonous and dicotyledonous plants. The test chemical was applied to one such flat immediately after it was seeded. The other flat contained plants on which the first true leaves had developed before the chemical was applied. The response was rated 12 to 21 days after treatment on a scale of 0 to 9 where 0 represents no injury and 9 represents complete kill.

The results are shown in the following tables:

TABLE I

Herbicidal Effectiveness of Post-Emergence Application (At 10 lbs./acre)

| | Phytotoxicity Rating* | | | | | |
|---|---|---|---|---|---|---|
| Compound of Example | MNGY | MSTD | FOX | JPN | CRB | PIG |
| 1 | 9 | 9 | 9 | 9 | 9 | 9 |
| 2 | 7 | 8 | 6 | 4 | 7 | 9 |
| 3 | 3 | 3 | 2 | 0 | 0 | 9 |

TABLE II

Herbicidal Effectiveness of Pre-Emergence Application (At 10 lbs./acre)

| | Phytotoxicity Rating* | | | | | |
|---|---|---|---|---|---|---|
| Compound of Example | MNGY | MSTD | FOX | JPN | CRB | PIG |
| 1 | 9 | 9 | 9 | 9 | 9 | 9 |
| 2 | 7 | 9 | 9 | 8 | 9 | 9 |
| 3 | 0 | 7 | 1 | 1 | 0 | 9 |

* Rated on scale of 0 to 9, from no visible effect on foliage to 100% destruction; MNGY = morning glory, MSTD = mustard, FOX = yellow foxtail, JPN = Japanese millet, CRB = crabgrass, PIG = pigweed.

EXAMPLE 75

Tests were made on two flats seeded with species of representative monocotyledonous and dicotyledonous plants. The test chemical was applied to one such flat immediately after it was seeded. The other flat contained plants on which the first true leaves had developed before the chemical was applied. The response was rated 12 to 21 days after treatment on a scale of 0 to 9 where 0 represents no injury and 9 represents complete kill.

The results are shown in the following tables:

TABLE III

Herbicidal Effectiveness of Post-Emergence Application (At 10 lbs./acre)

| Compound of Ex. No. | Phytotoxicity Rating* | | | | | |
|---|---|---|---|---|---|---|
| | MNGY | MSTD | FOX | JPN | CRB | PIG |
| 4 | 9 | 9 | 6 | 2 | 5 | 9 |
| 5 | 9 | 9 | 9 | 9 | 9 | 9 |
| 6 | 9 | 9 | 9 | 8 | 8 | 9 |
| 7 | 9 | 9 | 9 | 7 | 9 | 9 |
| 8 | 9 | 9 | 8 | 6 | 7 | 9 |
| 9** | 9 | 9 | 5 | 3 | 6 | 9 |
| 10 | 9 | 9 | 9 | 6 | 9 | 9 |
| 11 | 7 | 9 | 8 | 7 | 9 | 9 |
| 12 | 6 | 7 | 6 | 5 | 4 | 9 |
| 13 | 5 | 6 | 3 | 3 | 2 | 9 |
| 14** | 9 | 9 | 4 | 2 | 5 | 9 |
| 15 | 9 | 9 | 9 | 8 | 9 | 9 |
| 16 | 7 | 8 | 8 | 5 | 7 | 9 |
| 17 | 6 | 9 | 7 | 4 | 8 | 9 |
| 18 | $5^{N}$ | $7^{N}$ | $8^{N}$ | $6^{N}$ | $5^{N}$ | $8^{N}$ |
| 19 | 9 | 9 | $8^{S+}$ | $8^{S+}$ | 9 | 9 |
| 20 | 9 | 9 | 9 | 9 | 9 | 9 |
| 21 | 9 | 9 | 9 | 9 | 9 | 9 |
| 22 | $6^{S}$ | $8^{S}$ | $7^{S+}$ | 5 | $7^{S+}$ | 9 |
| 23 | $5^{S}$ | $4^{S}$ | 2 | 0 | 1 | 9 |
| 24 | $5^{S}$ | $7^{S+}$ | $8^{S+}$ | 4 | $8^{S}$ | 9 |

* Rated on scale of 0 to 9, from no visible effect on foliage to 100% destruction; with N = Necrosis; MNGY = morning glory, MSTD = mustard, FOX = yellow foxtail, JPN = Japanese millet, CRB = crabgrass, PIG = pigweed; S = moderate stunting, S+ = severe stunting.

** application rate 1.2 lbs./acre

TABLE III CONT'D

Herbicidal Effectiveness of Post-Emergence Application
(At 10 lbs./acre)

| Compound of Ex. No. | Phytotoxicity Rating* | | | | | |
|---|---|---|---|---|---|---|
| | MNGY | MSTD | FOX | JPN | CRB | PIG |
| 25 | $7^{S}$ | $8^{S+}$ | $6^{S}$ | 3 | $7^{S+}$ | $8^{S+}$ |
| 26 | 9 | 9 | 9 | 9 | 9 | 9 |
| 27 | 9 | 9 | 9 | 7 | 8 | 9 |
| 28 | 9 | 9 | 9 | 5 | 8 | 9 |
| 29 | 9 | 9 | 9 | 8 | 9 | 9 |
| 30 | 9 | 9 | 9 | 8 | 9 | 9 |
| 31 | 9 | 9 | 9 | 5 | 7 | 9 |
| 32 | 8 | 9 | 8 | 4 | 5 | 9 |
| 33 | 5 | 9 | 1 | 1 | 9 | 9 |
| 34 | 9 | 9 | 9 | 9 | 9 | 9 |
| 35 | 6 | 7 | 8 | 2 | 3 | 9 |
| 36 | $5^{S}$ | $4^{S}$ | 1 | 1 | 1 | 8 |
| 37 | $8^{S}$ | $8^{S}$ | $7^{S}$ | 0 | $6^{S}$ | 9 |
| 38 | | | | | | |
| 39 | 8 | 9 | 4 | $5^{S}$ | $3^{S}$ | 9 |
| 40 | 5 | 9 | 1 | 1 | 9 | 9 |
| 41 | 5 | 1 | 1 | 1 | 2 | 5 |
| 42 | 5 | 6 | 8 | 5 | 9 | 9 |
| 43 | 8 | 9 | 9 | 9 | 9 | 9 |

* Rated on scale of 0 to 9, from no visible effect on foliage to 100% destruction; with MNGY = morning glory, MSTD = mustard, FOX = yellow foxtail, JPN = Japanese millet, CRB = crabgrass, PIG = pigweed; S = moderate stunting, S+ = severe stunting.

TABLE IV

Herbicidal Effectiveness of Pre-Emergence Application
(At 10 lbs./acre)

| Compound of Ex. No. | Phytotoxicity Rating* | | | | | |
|---|---|---|---|---|---|---|
| | MNGY | MSTD | FOX | JPN | CRB | PIG |
| 4 | 9 | 9 | 9 | 9 | 9 | 9 |
| 5 | 9 | 9 | 9 | 9 | 9 | 9 |
| 6 | 9 | 9 | 9 | 8 | 9 | 9 |
| 7 | 9 | 9 | 9 | 9 | 9 | 9 |
| 8 | 8 | 9 | 7 | 5 | 9 | 9 |
| 9** | 9 | 9 | 6 | 5 | 9 | 9 |
| 10 | 9 | 9 | 9 | 9 | 9 | 9 |
| 11 | 9 | 9 | 9 | 8 | 9 | 9 |
| 12 | 7 | 9 | 8 | 2 | 8 | 9 |
| 13 | 5 | 9 | 9 | 7 | 8 | 9 |
| 14*** | 9 | 9 | $7^{S+}$ | $7^{S}$ | 9 | 9 |
| 15 | 9 | 9 | 9 | 7 | 9 | 9 |
| 16 | 9 | 9 | 9 | 6 | 9 | 9 |
| 17 | 7 | 9 | 9 | 6 | 9 | 9 |
| 18 | $4^{F}$ | $7^{S+,F}$ | 8 | $3^{C}$ | 8 | 9 |
| 19 | 9 | 9 | 9 | 9 | 9 | 9 |
| 20 | 9 | 9 | 9 | 9 | 9 | 9 |
| 21 | 9 | 9 | 9 | 9 | 9 | 9 |
| 22 | $7^{S+}$ | 9 | $8^{S+}$ | 5 | 9 | 9 |
| 23 | 9 | 9 | $4^{S,F}$ | 3 | $4^{S}$ | 9 |
| 24 | 9 | 9 | $8^{S+}$ | 9 | 9 | 9 |

* Rated on scale of 0 to 9, from no visible effect on foliage to 100% destruction; with S = moderate stunting, S+ = severe stunting, C = chlorosis, F = formative; MNGY = morning glory, MSTD = mustard, FOX = yellow foxtail, JPN = Japanese millet, CRB = crabgrass, PIG = pigweed.

** application rate 1.2 lbs./acre

*** application rate 2.0 lbs./acre

TABLE IV CONT'D

Herbicidal Effectiveness of Pre-Emergence Application (At 10 lbs./acre)

| Compound of Ex. No. | Phytotoxicity Rating* | | | | | |
|---|---|---|---|---|---|---|
| | MNGY | MSTD | FOX | JPN | CRB | PIG |
| 25 | 9 | 9 | 9 | $6^{S}$ | 9 | 9 |
| 26 | 9 | 9 | 9 | 9 | 9 | 9 |
| 27 | 9 | 9 | 9 | 9 | 9 | 9 |
| 28 | 9 | 9 | 9 | 9 | 9 | 9 |
| 29 | 9 | 9 | 9 | 8 | 9 | 9 |
| 30 | 9 | 9 | 9 | 9 | 9 | 9 |
| 31 | 5 | 9 | 8 | 6 | 9 | 9 |
| 32 | 9 | 9 | 6 | 8 | 9 | 9 |
| 33 | 5 | 9 | $5^{S}$ | 1 | $8^{S+}$ | 9 |
| 34 | 9 | 7 | 9 | 9 | 9 | 9 |
| 35 | 8 | 7 | 7 | 5 | 2 | 9 |
| 36 | $6^{S}$ | $7^{S}$ | $4^{S}$ | $3^{S}$ | $5^{S}$ | 9 |
| 37 | 9 | 9 | 9 | 9 | 9 | 9 |
| 38 | | | | | | |
| 39 | 8 | 9 | $7^{S,F}$ | 8 | 9 | 9 |
| 40 | 5 | 9 | $5^{S}$ | 1 | $8^{S+}$ | 9 |
| 41 | 5 | $8^{S+}$ | $5^{S+}$ | 5 | $7^{S+}$ | 9 |
| 42 | 9 | 9 | 9 | 9 | 9 | 9 |
| 43 | 9 | 9 | 9 | 9 | 9 | 9 |

* Rated on scale of 0 to 9, from no visible effect on foliage to 100% destruction; with S = moderate stunting, S+ = severe stunting, C = chlorosis, F = formative; MNGY = morning glory, MSTD = mustard, FOX = yellow foxtail, JPN = Japanese millet, CRB = crabgrass, PIG = pigweed.

EXAMPLE 76

Tests were made on two flats seeded with species of representative monocotyledonous and dicotyledonous plants. The test chemical was applied to one such flat immediately after it was seeded. The other flat contained plants on which the first true leaves had developed before the chemical was applied. The response was rated 12 to 21 days after treatment on a scale of 0 to 9 where 0 represents no injury and 9 represents complete kill.

The results are shown in the following tables:

TABLE V

Herbicidal Effectiveness of Pre-Emergence Application (At 10 lbs./acre)

| Compound of | | | Phytotoxicity Rating* | | | |
|---|---|---|---|---|---|---|
| Ex. No. | MNGY | MSTD | FOX | JPN | CRB | PIG |
| 44 | 9 | 9 | 9 | 9 | 9 | 9 |
| 45 | 9 | 9 | 8 | 7 | 9 | 9 |

TABLE VI

Herbicidal Effectiveness of Post-Emergence Application (At 10 lbs./acre)

| Compound of | | | Phytotoxicity Rating* | | | |
|---|---|---|---|---|---|---|
| Ex. No. | MNGY | MSTD | FOX | JPN | CRB | PIG |
| 44 | 7 | 9 | 7 | $5^F$ | 8 | 9 |
| 45 | 9 | 9 | 9 | 8 | 9 | 9 |

* Rated on scale of 0 to 9, from no visible effect on foliage to 100% destruction; with F = formative; MNGY = morning glory, MSTD = mustard, FOX = yellow foxtail, JPN = Japanese millet, CRB = crabgrass, PIG = pigweed.

EXAMPLE 77

Tests were made on two flats seeded with species of representative monocotyledonous and dicotyledonous plants. The test chemical was applied to one such flat immediately after it was seeded. The other flat contained plants on which the first true leaves had developed before the chemical was applied. The response was rated 12 to 21 days after treatment on a scale of 0 to 9 where 0 represents no injury and 9 represents complete kill.

The results are shown in the following tables:

TABLE VII

Herbicidal Effectiveness of Post-Emergence Application (at 10 lbs./acre)

| Compound of Example | Phytotoxicity Rating* MNGY | MSTD | FOX | JPN | CRB | PIG |
|---|---|---|---|---|---|---|
| 46 | 5 | 8 | 3 | 2 | 4 | 7 |
| 47 | 2 | 4 | 4 | 0 | 0 | 9 |
| 48 | 7 | 9 | 8 | 6 | 7 | 9 |
| 49 | 8 | 9 | 9 | 5 | 8 | 9 |
| 50 | 7 | 9 | 9 | 5 | 9 | 9 |
| 51 | 4 | 6 | 7 | 4 | 5 | 9 |
| 52 | 9 | 9 | 5 | 1 | 9 | 9 |
| 53 | 4 | 5 | 2 | 1 | 2 | 9 |
| 54 | 2 | 4 | 4 | 0 | 0 | 9 |
| 55 | 3 | 4 | 1 | 0 | 3 | 5 |
| 56 | 2F | 4 | 1 | 1 | 1 | 9 |
| 57 | 0 | 0 | 0 | 0 | 0 | 0 |
| 58 | 2 | 6E | 1 | 2 | 0 | 6 |
| 59 | 9 | 9 | 9 | 9 | 9 | 9 |
| 60 | 2 | 2 | 2 | 2 | 2 | 5 |

* F = formative; E = epinasty; MNGY = morning glory, MSTD = mustard, FOX = yellow foxtail, JPN = Japanese millet, CRB = crabgrass, PIG = pigweed.
Rated on scale of 0 to 9 as in Table VI.

TABLE VIII

Herbicidal Effectiveness of Pre-Emergence Application (at 10 lbs./acre)

| Compound of Example | MNGY | Phytotoxicity Rating* MSTD | FOX | JPN | CRB | PIG |
|---|---|---|---|---|---|---|
| 46 | 5 | 9 | 6 | 3 | 4 | 5 |
| 47 | 2 | 7 | 5 | 6 | 9 | 9 |
| 48 | 9 | 9 | 7 | 6 | 9 | 9 |
| 49 | 9 | 9 | 5 | 2 | 0 | 0 |
| 50 | 9 | 9 | 7 | 6 | 9 | 9 |
| 51 | 3 | 9 | 7 | 5 | 8 | 9 |
| 52 | 5 | 9 | $6^{S,F}$ | 3 | 9 | 9 |
| 53 | 0 | $7^{S+}$ | $5^{F}$ | $3^{F}$ | $7^{S+}$ | 9 |
| 54 | 2 | 7 | 5 | 6 | 9 | 9 |
| 55 | 5 | 9 | 2 | 1 | $7^{S}$ | 9 |
| 56 | 1 | $8^{S}$ | 2 | 2 | $6^{S+}$ | 9 |
| 57 | 1 | 0 | 0 | 0 | 0 | $1^{S}$ |
| 58 | 0 | $5^{S}$ | $2^{E}$ | $3^{F}$ | $6^{S+}$ | 9 |
| 59 | 9 | 9 | 9 | 9 | 9 | 9 |
| 60 | 2 | 4 | 1 | 2 | $5^{S,F}$ | 9 |

* Rated on scale of 0 to 9, from no visible effect on foliage to 100% destruction; with MNGY = morning glory, MSTD = mustard, FOX = yellow foxtail, JPN = Japanese millet, CRB = crabgrass, PIG = pigweed; F = formative, S = moderate stunting and S+ = severe stunting.

EXAMPLE 78

Tests were made on two flats seeded with species of representative monocotyledonous and dicotyledonous plants. The test chemical was applied to one such flat immediately after it was seeded. The other flat contained plants on which the first true leaves had developed before the chemical was applied. The response was rated 12 to 21 days after treatment on a scale of 0 to 9 where 0 represents no injury and 9 represents complete kill.

TABLE IX

Herbicidal Effectiveness of Pre-Emergence Application (At 10 lbs./acre)

| Compound of Ex. No. | Phytotoxicity Rating* | | | | | |
|---|---|---|---|---|---|---|
| | MNGY | MSTD | FOX | JPN | CRB | PIG |
| 61 | 9 | 9 | 7 | 4 | 5 | 9 |
| 62 | 9 | 9 | 9 | 5 | 9 | 9 |
| 63 | 8 | 9 | 7 | 4 | 8 | 9 |
| 64 | 6 | 9 | 9 | 5 | 9 | 9 |

TABLE X

Herbicidal Effectiveness of Post-Emergence Application (At 10 lbs./acre)

| Compound of Ex. No. | Phytotoxicity Rating* | | | | | |
|---|---|---|---|---|---|---|
| | MNGY | MSTD | FOX | JPN | CRB | PIG |
| 61 | 9 | 9 | 9 | 4 | 9 | 9 |
| 62 | 9 | 9 | 8 | 8 | 9 | 9 |
| 63 | 7 | 8 | 6 | 5 | 6 | 9 |
| 64 | 9 | 9 | 9 | 9 | 9 | 9 |

* Rated on scale of 0 to 9, from no visible effect on foliage to 100% destruction; MNGY = morning glory, MSTD = mustard, FOX = yellow foxtail, JPN = Japanese millet, CRB = crabgrass, PIG = pigweed.

EXAMPLE 79

The following tests were carried out as described in Example 78.

TABLE XI

Herbicidal Effectiveness of Post-Emergence Application (at 10 lbs./acre)

| Compound of Example No. | Phytotoxicity Rating* | | | | | |
|---|---|---|---|---|---|---|
| | MNGTY | MSTD | FOX | JPN | CRB | PIG |
| 65 | $7^{S+,F}$ | $7^{S+,F}$ | 3 | $5^{S,F}$ | $6^{S+}$ | 9 |
| 66 | $4^{F+,S}$ | $5^{E,S}$ | $7^{N}$ | $6^{N,S}$ | $4^{N}$ | $8^{N}$ |
| 67 | 9 | 9 | 9 | $8^{S+}$ | 9 | 9 |
| 68 | 9 | 9 | 9 | 8 | 8 | 9 |
| 69 | 9 | 9 | 9 | $8^{S+}$ | 9 | 9 |
| 70 | 9 | 9 | 9 | 9 | 9 | 9 |

TABLE XII

Herbicidal Effectiveness of Pre-Emergence Application (at 10 lbs./acre)

| Compound of Example No. | Phytotoxicity Rating* | | | | | |
|---|---|---|---|---|---|---|
| | MNGTY | MSTD | FOX | JPN | CRB | PIG |
| 65 | 9 | $8^{S+}$ | 1 | 2 | 1 | 9 |
| 66 | $7^{E,N}$ | 9 | 9 | 9 | 9 | 9 |
| 67 | 9 | 9 | 9 | 9 | 9 | 9 |
| 68 | 9 | 9 | 7 | 9 | 9 | 9 |
| 69 | 9 | 9 | 9 | 9 | 9 | 9 |
| 70 | 9 | 9 | 9 | 9 | 9 | 9 |

* Rated on scale of 0 to 9, from no visible effect on foliage to 100% destruction; with S = moderate stunting, S+ = severe stunting, F = formative, E = epinasty, N = Necrosis; MNGY = morning glory, MSTD = mustard, FOX = yellow foxtail, JPN = Japanese millet, CRB = crabgrass, PIG = pigweed.

In summary, the compounds of this invention show high pre- and post- emergence herbicidal activity against indicator weeds, and are particularly effective against broadleaf weeds, such as morning glory. In addition to such effective herbicidal activity, they exhibit an unusual selectivity against important agromonic crops, such as cotton, rice, soybean, corn, wheat and sorghum.

While the invention has been described with particular reference to certain embodiments thereof, it will be understood that certain modifications and changes may be made which are within the skill of the art. Therefore it is intended to be bound only by the appended claims.

WHAT IS CLAIMED IS:

1. A . compound having the formula:

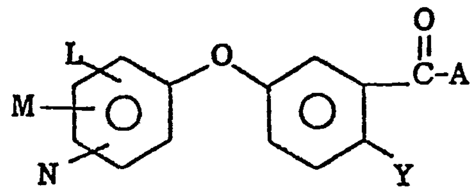

1.

wherein L, M and N are independently hydrogen, hydroxy, halogen, trihalomethyl, nitro, cyano, $C_{1-4}$ alkyl, $C_{1-4}$ alkoxy and 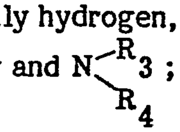 ; Y represents nitro, cyano, or 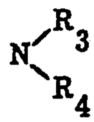 and A is selected from the group of radicals defined by the formulae:

I $-Z(CH_2CH_2Z')_mCH_2CH_2Z''R_7$;

II 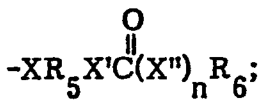

III $-X(CH_2)_nR_6(OH)_p$;

IV 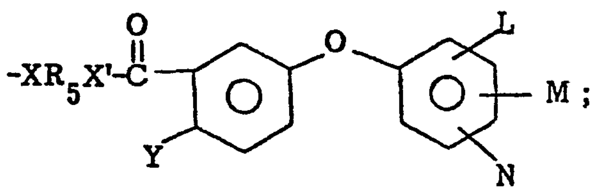

V 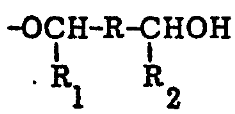 and ,

VI 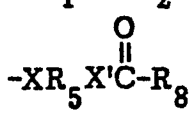

wherein

R is an unsaturated, straight chain or branched aliphatic radical having from 2 to 8 carbon atoms;

$R_1$ and $R_2$ are independently hydrogen or alkyl of 1 to 4 carbon atoms;

$R_3$ and $R_4$ are independently hydrogen, or a saturated or unsaturated straight or branched chain $C_{1-8}$ aliphatic radical optionally substituted with halogen, hydroxy, alkoxy, cyano or nitro;

$\underline{R_5}$ is a saturated or unsaturated, straight chain or branched aliphatic hydrocarbon radical of from 1 to 18 carbon atoms wherein one or more of the $-CH_2-$ groups can be replaced with -O-, -S-, -S-S-, -SO-, $-SO_2-$ or $-NR_4-$ and said hydrocarbon radical is optionally substituted with halogen, trihalomethyl, cyano, aryl, hydroxy, alkoxy, nitro or cycloalkyl having 3 to 6 carbon atoms;

$\underline{R_6}$ is a saturated or unsaturated straight chain or branched aliphatic radical containing from 1 to 8 carbon atoms, optionally substituted with halogen, trihalomethyl, cyano, hydroxy, nitro, acetoxy, alkoxy, thioalkoxy or aryl; an aryl radical optionally substituted with halogen, trihalomethyl, hydroxy, cyano, nitro, alkyl or alkoxy; a cyclic 3-6 membered alkylene ring or 5-6 membered alkenylene ring or benzyl optionally substituted with halogen, trihalomethyl, alkyl, hydroxy, alkoxy or cyano;

$\underline{R_7}$ is hydrogen, $C_{1-4}$ alkyl optionally substituted with halogen, trihalomethyl or cyano or aryl optionally substituted with halogen, trihalomethyl, cyano, $C_{1-4}$ alkoxy or nitro;

$\underline{R_8}$ is $-\overset{O}{\overset{\|}{C}}-(O)_nR_6$, $-R_9-\overset{O}{\overset{\|}{C}}-(OR_9)_n(O)_nR_6$ or $-R_9-O-$ 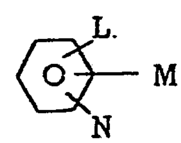 ;

each $\underline{R_9}$ is independently an alkylene diradical having from 1 to 4 carbon atoms;

<u>X, X' and X"</u> are independently -O-, -S- or $-NR_4-$;

<u>Z and Z"</u> are independently, -O- or -S-, Z" additionally can be thiol or $-SO_3-$, and Z' is -S-, -S-S-, -SO- or $-SO_2-$;

$\underline{p}$ has a value of 2-6; $\underline{n}$ in each instance, has a value of 0 or 1; and $\underline{m}$ has a value of 1-6.

2. The compound of Claim 1 having the formula:

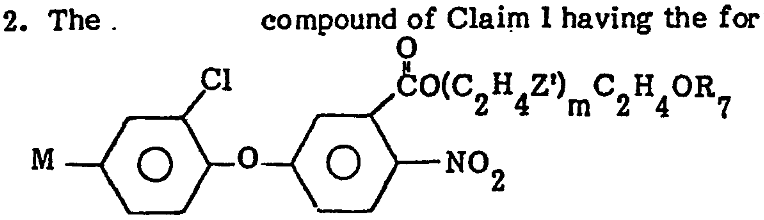

wherein M is $-CF_3$, -Cl or $-CH_3$

Z' is -S-, -S-S- or $-SO_2-$

$R_7$ is -H or a lower alkyl radical

m is 1-3.

3. The compound of Claim 1 having the formula:

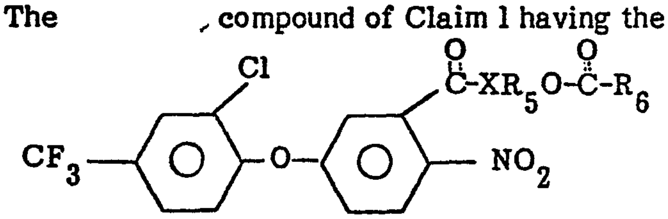

wherein X is -O-, -S- or $-N(CH_3)-$

$R_5$ is an alkylene diradical of 2 to 6 carbon atoms or

$-(CH_2CH_2O)_{1-5}CH_2CH_2-$

$R_6$ is dialkylamino; alkyl having from 1 to 6 carbon atoms, optionally substituted with halogen; or cyclic alkyl having 3 to 6 carbon atoms.

4. The compound of Claim 1 having the formula:

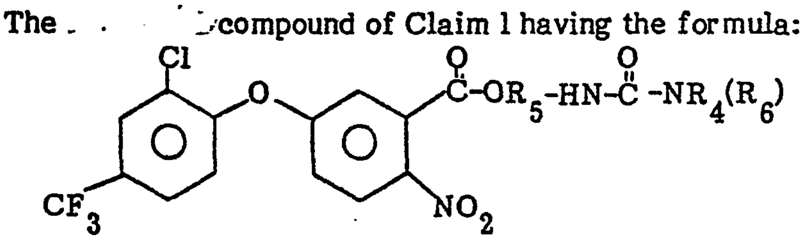

wherein $R_6$ and $R_4$ are independently hydrogen or alkyl of 1 to 4 carbon atoms and

$R_5$ is an alkylene diradical of 2 to 6 carbon atoms or

$-(CH_2CH_2O)_{1-5}CH_2CH_2-$.

5. The compound of Claim 1 having the formula:

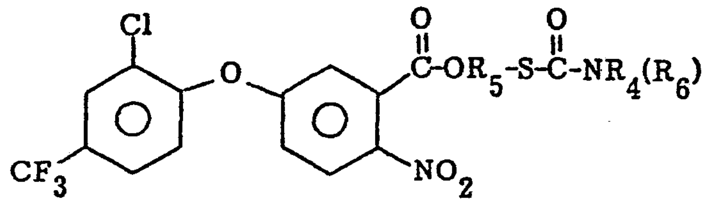

wherein $R_6$ and $R_4$ are independently hydrogen or alkyl of 1 to 4 carbon atoms and

$R_5$ is an alkylene diradical of 2 to 6 carbon atoms or

$-(CH_2CH_2O)_{1-5}CH_2CH_2-$.

6. The compound of Claim 1 having the formula:

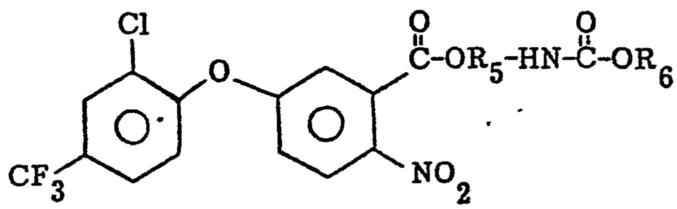

wherein $R_5$ is an alkylene diradical of 2 to 6 carbon atoms or

$-(CH_2CH_2O)_{1-5}CH_2CH_2-$ and

$R_6$ is an alkyl radical of 1 to 4 carbon atoms.

7. The compound of Claim 1 having the formula:

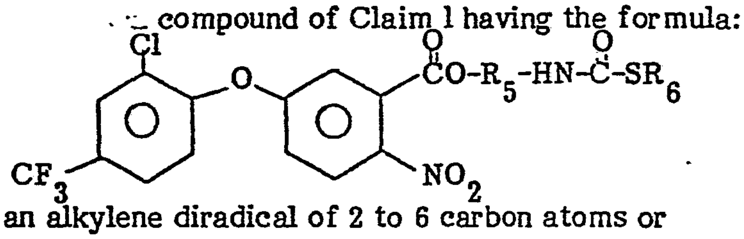

wherein $R_5$ is an alkylene diradical of 2 to 6 carbon atoms or

$-(CH_2CH_2O)_{1-5}CH_2CH_2-$ and

$R_6$ is an alkyl radical of 1 to 4 carbon atoms.

8. The compound of Claim 1 having the formula:

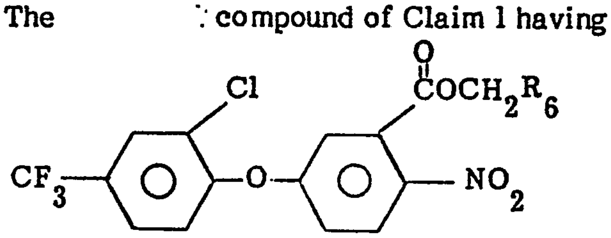

wherein $R_6$ is a polyhydroxylated alkylene having from 2 to 4 carbon atoms.

9. The compound of Claim 1 having the formula:

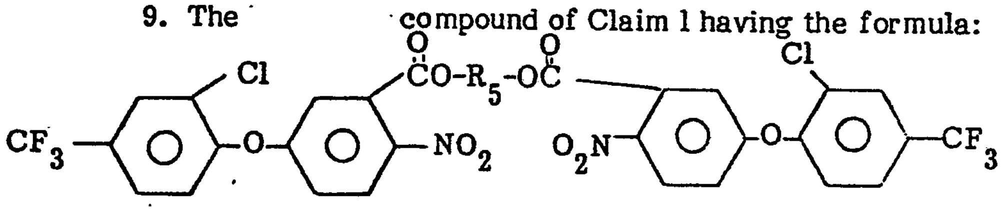

wherein $R_5$ is alkylene having from 2 to 10 carbon atoms or $-(CH_2CH_2Z)_mCH_2CH_2-$ where m is 1 to 5 and Z is oxygen or sulfur.

10. The compound having the formula:

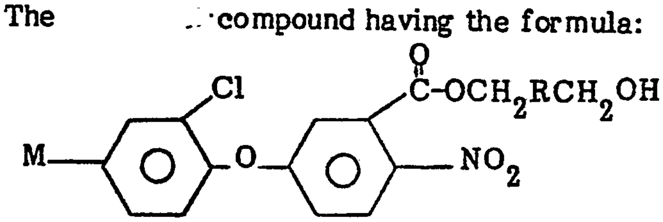

wherein M is $-CF_3$ or $-Cl$ and R contains from 2 to 4 carbon atoms and is an aliphatically unsaturated hydrocarbon radical.

11. The compound of Claim 1 having the formula:

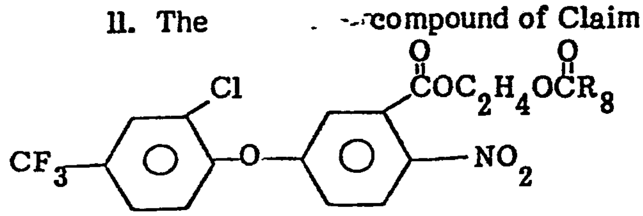

wherein $R_8$ is 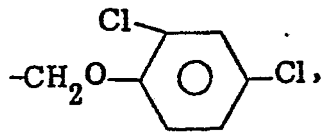, $-(CH_2)_m\overset{O}{\overset{\|}{C}}O-$ lower alkyl or $-\overset{O}{\overset{\|}{C}}(O)_n$ lower alkyl.

12. The compound of Claim 1 which is [2-(chloroacetoxy)ethyl] 5-(2-chloro-4-trifluoromethylphenoxy)-2-nitrobenzoate.

13. The compound of Claim 1 which is [2-acetoxyethyl] 5-(2-chloro-4-trifluoromethylphenoxy)-2-nitrobenzoate.

14. The compound of Claim 1 which is [2-(propionyloxy)ethyl] 5-(2-chloro-4-trifluoromethylphenoxy)-2-nitrobenzoate.

15. The compound of Claim 1 which is [2-(methoxyacetoxy)ethyl] 5-(2-chloro-4-trifluoromethylphenoxy)-2-nitrobenzoate.

16. The compound of Claim 1 which is [2-(N,N-diethylcarbamyloxy)ethyl] 5-(2-chloro-4-trifluoromethylphenoxy)-2-nitrobenzoate.

17. The compound of Claim 1 which is [4-(N,N-dimethylcarbamyloxy)-2-buten-1-yl] 5-(2-chloro-4-trifluoromethylphenoxy)-2-nitrobenzoate.

18. A herbicidal composition comprising an effective amount of a compound of the formula of Claim 1 and an agronomically acceptable carrier.

19. A method of controlling undesirable plant growth which comprises applying to the undesirable plant, plant part or plant situs a growth controlling amount of the herbicidal compound of Claim 1.

20. The method of Claim 19 wherein said application of said herbicide is effected during the growth stage of a desirable crop.

21. The method of Claim 19 wherein the herbicidal compound is applied at a rate of from 0.05 to 8 pounds per acre.

22. The method of Claim 21 wherein the herbicidal compound in an inert liquid carrier is applied as a liquid spray.

23. The process for the preparation of substituted diphenyl ethers of Claim 1 having the formula:

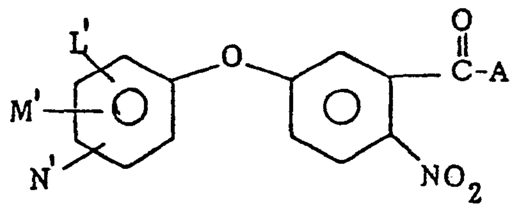

wherein L,' M' and N' are independently hydrogen, halogen, trihalomethyl, nitro, cyano, $C_{1-4}$ alkyl, $C_{1-4}$ alkoxy and $N<^{R_3}_{R_4}$ ;

which comprises reacting a correspondingly substituted phenoxybenzoic acid having the formula

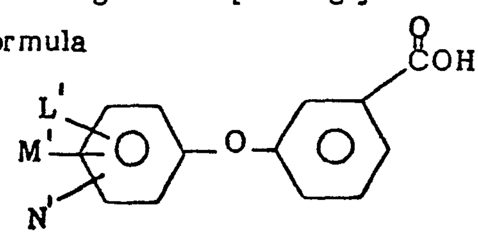

with about a stoichiometric amount of a diacylating agent having the formula HX-A'-X'H where A' is $R_5$, $-\underset{R_1}{CH}-(R)_n-\underset{R_2}{CH}-$ or $-(CH_2)_nR_6(OH)_{p-1}$ to produce the corresponding bis compound and then nitrating the bis compound to produce a compound having the formula

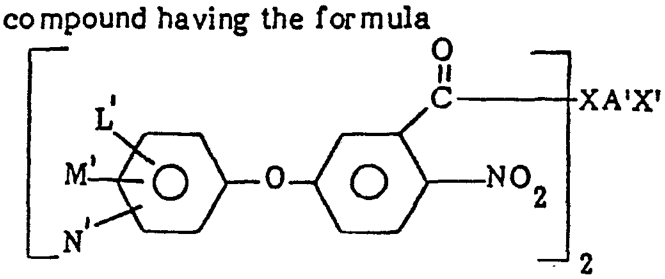

;

and then optionally transacylating the bis compound with an excess of said diacylating agent, preferably in the presence of a transacylating catalyst, to produce the compounds of formula 1 where A is I, III or V, namely

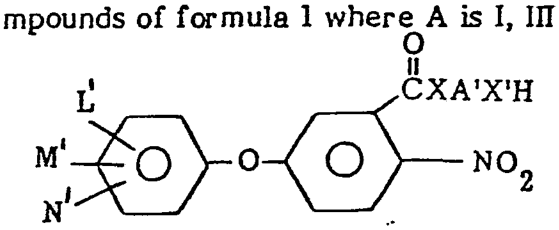

and further optionally reacting the product of transacylation where A' is $R_5$ with a carbonyl-containing compound of the formula

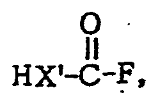

or the acid halide or anhydride of such carbonyl-containing compounds to produce the compounds of formula 1 where A is II or VI, namely

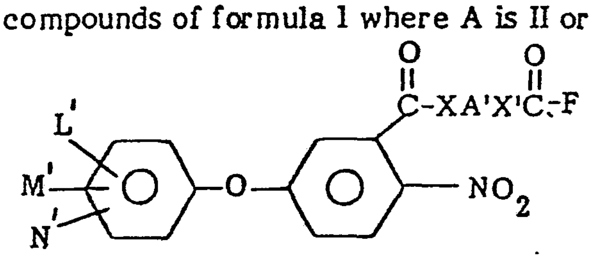

where F is $R_8$ or $(X'')_nR_6$.

24. The process for the preparation of substituted diphenyl ethers of Claim 23 wherein A is group IV which comprises reacting a phenoxybenzoic acid having the formula

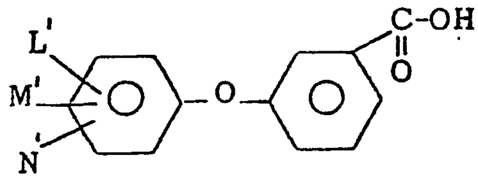

with a diacylating agent having the formula HX-A'-X'H wherein A' is $R_5$, $-\underset{R_1}{CH}-(R)_n-\underset{R_2}{CH}-$ or $-(CH_2)_nR_6(OH)_{p-1}$ to produce the corresponding bis intermediate compound having the formula:

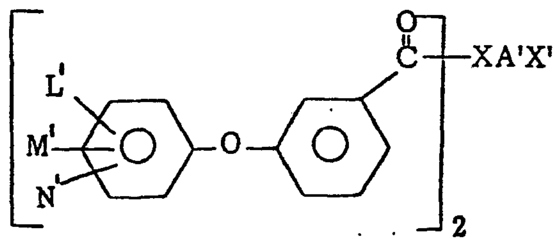

and then nitrating said bis compound with a nitrating agent to produce the product having the formula:

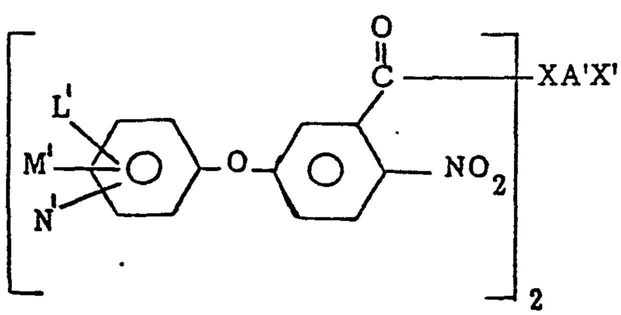

25. The process for the preparation of substituted diphenyl ethers of Claim 23 wherein A is group I, III or V which comprises transacylating the product of Claim 24 with an excess of said diacylating agent in the presence of a transacylating catalyst in a transacylation zone to produce the corresponding product having the formula:

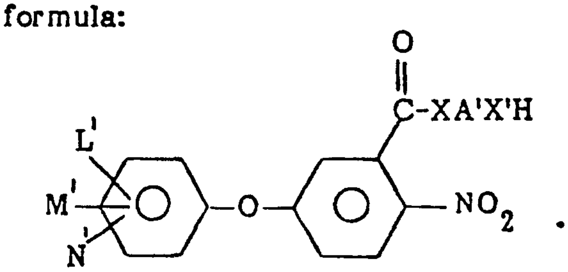

26. The process for the preparation of substituted diphenyl ethers of Claim 23 wherein A is group II or VI which comprises reacting the product of Claim 25 where A' is $R_5$ with a carbonyl-containing compound selected from the group of a compound having the formula $HX'-\overset{O}{\overset{\|}{C}}-F$ and the acid halide or anhydride of said compound wherein F is $R_8$ or $(X'')_nR_6$, to produce the product having the formula:

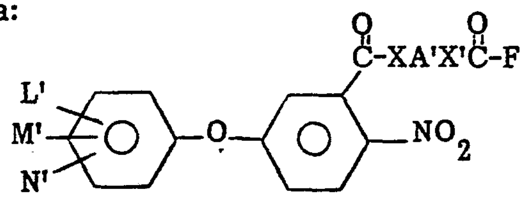

27. The process of Claim 24 wherein the reaction of said phenoxybenzoic acid with said diacylating agent is effected at a temperature between about $20^{\circ}$ and about $200^{\circ}$C. under from about 1 to about 5 atmospheres pressure and the nitration of said intermediate is effected at a temperature between about $-5^{\circ}$ and about $70^{\circ}$C. under atmospheric pressure.

28. The process of Claim 27 wherein the nitration is effected in the presence of an inert solvent and the concentration of said intermediate compound in said solvent is between about 10 and about 80 weight percent.

29. The process of Claim 27 wherein the mole ratio of said phenoxybenzoic acid to said diacylating agent is about 2:1 and the mole ratio of nitrating agent to said intermediate compound is about 2:1.

30. The process of Claim 24 wherein said phenoxybenzoic acid is 3-(2-chloro-4-trifluoromethylphenoxy)benzoic acid.

31. The process of Claim 30 wherein said diacylating agent is ethylene glycol.

32. The process of Claim 30 wherein said diacylating agent is 1,3-propandiol.

33. The process of Claim 30 wherein said diacylating agent is 2-hydroxyethyl ether.

34. The process of Claim 30 wherein said diacylating agent is 2-hydroxyethyl sulfide.

35. The process of Claim 25 wherein said transacylation reaction is effected at a temperature between about 20$^{o}$ and about 250$^{o}$C. under from about 1 to about 5 atmospheres pressure.

36. The process of Claim 35 wherein the catalyst is manganese acetate tetrahydrate.

37. The process of Claim 35 wherein the catalyst is an acid.

38. The process of Claim 35 wherein the mole ratio of diacylating agent to nitrated product is between about 1:1 and about 50:1 and unreacted diacylating agent is recycled to said transacylation zone.

39. The process of Claim 35 wherein said diacylating agent is ethylene glycol.

40. The process of Claim 39 wherein 1,2-bis[5-(2-chloro-4-trifluoromethylphenoxy)-2-nitrobenzoyloxy]ethane is reacted with ethylene glycol.

41. The process of Claim 35 wherein 1,3-bis[5-(2-chloro-4-trifluoromethylphenoxy)-2-nitrobenzoyloxy]propane is reacted with 1,3-propanediol.

42. The process of Claim 35 wherein 1,5-bis[5-(2-chloro-4-trifluoromethylphenoxy)-2-nitrobenzoyloxy]-3-oxapentane is reacted with 2-hydroxyethyl ether.

43. The process of Claim 35 wherein 1,5-bis[5-(2-chloro-4-trifluoromethylphenoxy)-2-nitrobenzoyloxy]-3-thiapentane is reacted with 2-hydroxyethyl sulfide.

44. The process of Claim 35 wherein 1,4-bis[5-(2-chloro-4-trifluoromethylphenoxy)-2-nitrobenzoyloxy]-2-butene is reacted with 1,4-butenediol.

45. The process of Claim 26 wherein said reaction with said carbonyl-containing compound is effected at a temperature of between about $0^{o}$ and about $200^{o}C$. under from about 1 to about 5 atmospheres pressure.

46. The process of Claim 45 wherein the mole ratio of said carbonyl-containing compound to said transacylated component is between about 1:1 and about 20:1.

47. The process of Claim 26 wherein [2-hydroxyethyl] 5-(2-chloro-4-trifluoromethylphenoxy)-2-nitrobenzoate is reacted with a carbonyl-containing compound.

48. The process of Claim 47 wherein the carbonyl-containing compound is acetic acid.

49. The process of Claim 47 wherein the carbonyl-containing compound is chloroacetic acid.

50. The process of Claim 47 wherein the carbonyl-containing compound is methoxyacetic acid.

51. The process of Claim 47 wherein the carbonyl-containing compound is propionic acid.

0066106

European Patent Office

# EUROPEAN SEARCH REPORT

Application number

EP 82 10 3897

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,E | EP-A-0 040 898 (MOBIL OIL CORP.)<br>* Pages 11,12, compound 2; page 15, claim 1; page 17, claim 18; page 8, last paragraph *<br>--- | 1,18, 19-22 | C 07 C 79/46<br>C 07 C 149/40<br>C 07 C 101/52<br>C 07 C 103/46<br>C 07 C 125/06<br>C 07 C 153/09<br>A 01 N 47/10<br>A 01 N 37/10<br>A 01 N 33/22 |
| X | EP-A-0 028 277 (ROHM AND HAAS)<br>* Page 2, line 20 - page 5, line 25 *<br>--- | 1,18-22 | |
| X | EP-A-0 021 692 (ROHM AND HAAS)<br>* Page 2, line 11 - page 6, line 2; page 7, line 24 - page 8, line 28 *<br>--- | 1,19-22 | |
| P,E | EP-A-0 030 676 (BAYER)<br>* Page 21, lines 6-11; page 23, line 24 - page 24, line 9; pages 32-33; claim 1 *<br>----- | 1,19-22 | **TECHNICAL FIELDS SEARCHED (Int. Cl. 3)**<br>C 07 C 79/00<br>C 07 C 69/00 |

The present search report has been drawn up for all claims

| Place of search | Date of completion of the search | Examiner |
|---|---|---|
| THE HAGUE | 27-08-1982 | KINZINGER J.M. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82